# FASCICULE DE BREVET EUROPEEN

(11) **EP 2 793 681 B1**
(45) Date de publication et mention de la délivrance du brevet: **17.10.2018**
(21) Numéro de dépôt: 12816759.0
(22) Date de dépôt: 20.12.2012
(51) Int. Cl.: A61B 3/00, A61B 3/103, G02C 7/02, A61B 3/18

(54) **DISPOSITIF ET PROCÉDÉ DE DÉTERMINATION D'AU MOINS UN PARAMÈTRE DE RÉFRACTION OCULAIRE OBJECTIVE D'UN SUJET EN FONCTION D'UNE PLURALITÉ DE DIRECTIONS DE REGARD**
VORRICHTUNG UND VERFAHREN ZUR BESTIMMUNG MINDESTENS EINES OBJEKTIVBRECHUNGSPARAMETERS EINER PERSON IN ABHÄNGIGKEIT VON MEHREREN BLICKRICHTUNGEN
DEVICE AND METHOD FOR DETERMINING AT LEAST ONE OBJECTIVE EYE REFRACTION PARAMETER OF A SUBJECT DEPENDING ON A PLURALITY OF GAZE DIRECTIONS

(30) Priorité: 22.12.2011 FR 1104035
(43) Date de publication de la demande: 29.10.2014
(73) Titulaire: Essilor International, 94220 Charenton-le-Pont (FR)
(72) Inventeur: BARANTON, Konogan, F-94220 Charenton-Le-Pont (FR); DIVO, Fabien, F-94220 Charenton-Le-Pont (FR); ESCALIER, Guilhem, F-94220 Charenton-Le-Pont (FR); HERNANDEZ-CASTANEDA, Martha, F-94220 Charenton-Le-Pont (FR); MARIN, Gildas, F-94220 Charenton-Le-Pont (FR); OURIVES, Pedro, F-94220 Charenton-Le-Pont (FR); ROUSSEAU, Benjamin, F-94220 Charenton-Le-Pont (FR)
(74) Mandataire: Jacobacci Coralis Harle
(86) Numéro de dépôt international: PCT/FR2012/053033
(87) Numéro de publication internationale: WO 2013/093363

(56) Documents cités:
- US-A- 2 635 502
- US-A1- 2005 018 132
- US-A1- 2005 174 536

## Description

### DOMAINE TECHNIQUE AUQUEL SE RAPPORTE L'INVENTION

La présente invention concerne de manière générale le domaine des dispositifs et procédés d'optométrie. Plus particulièrement, l'invention concerne un appareil d'optométrie pour déterminer les différentes valeurs de la prescription d'une lentille de lunettes de compensation visuelle multifocale ou progressive, ou de lunettes destinées à la compensation en vision de près (readers), liées à la mesure de la réfraction oculaire différenciée d'une pluralité de regards et en particulier en vision de loin et en vision de près. Ces mesures sont destinées à être exploitées pour la conception optique et la fabrication des faces de réfraction d'une lentille correctrice de lunettes multifocale ou progressive, ou destinées à la compensation en vision de près (readers, pré-montées), que ce soit des verres passifs ou de puissances optiques variables par commande électronique.

### ARRIERE-PLAN TECHNOLOGIQUE

Depuis une cinquantaine d'années, les lentilles de lunettes multifocales et progressives ont connu un essor considérable. Une lentille multi-focale présente au moins deux puissances correctives distinctes pour deux zones de la lentille correspondant à deux distances de vision. Une lentille progressive présente une puissance variable sur la surface de la lentille, allant, par exemple dans le cas d'une compensation de presbytie, d'une zone où la puissance sphérique est faible pour la vision de loin (VL) à une zone où la puissance sphérique est plus forte pour la vision de près (VP). Une lentille progressive présente généralement une compensation moyenne pour une distance de vision intermédiaire entre la vision de loin et la vision de près. Les lentilles de lunettes multifocales ou progressives permettent au porteur de bénéficier d'une compensation de puissance optique adaptée pour différentes distances de vision sans changer de lunettes. Afin de déterminer les paramètres d'une lentille multi-focale ou d'une lentille progressive, il existe des appareils d'optométrie monoculaires ou binoculaires pour mesurer la compensation optique à apporter en vision de près et en vision de loin, dans lesquels l'axe de visée du regard reste horizontal quelle que soit la distance de visée. Un appareil d'optométrie basé sur la mesure de la réfraction d'un faisceau lumineux sur un oeil permet ainsi de mesurer la compensation de puissance (ou sphère) différenciée VL/VP c'est-à-dire les compensations à apporter à l'oeil mesuré en vision de près et en vision de loin. Une lentille multi-focale ou progressive peut compenser non seulement un défaut de puissance optique mais aussi d'autres défauts de vision et en particulier l'astigmatisme. Basé sur le même principe de mesure de réfraction oculaire, la plupart des appareils d'optométrie permettent de mesurer les paramètres de compensation d'astigmatisme (cylindre et axe) et/ou les paramètres de compensation des aberrations d'ordres supérieur (cf. norme ISO 24157 :2008 qui spécifie les méthodes normalisées permettant de consigner les aberrations de l'oeil humain).

Actuellement les mesures de réfraction oculaire différenciées VL/VP se font uniquement de façon manuelle. Un optométriste utilise une lunette d'essai pour déterminer les différentes valeurs de la prescription des lentilles.

Dans les appareils classiques d'optométrie, un système optique intercalé sur l'axe oculaire adapte la puissance optique pour modifier la distance d'accommodation visuelle sur une cible, la ligne de visée du regard restant horizontale.

Le document US2005/0174536 décrit un appareil pour examiner l'accommodation oculaire d'un sujet en fonction du changement de valeur de proximité d'une cible sans modification de l'angle d'abaissement du regard.

Le document US5635502 décrit un appareil pour tester la tonicité des muscles oculaires d'un sujet dans des conditions de vision indépendantes pour chacun des deux yeux du sujet.

Dans les études actuelles sur la mesure de réfraction différenciée (VL/VP), nous sommes confrontés au problème de mesurer la réfraction en vision de près tout en suivant l'abaissement physiologique du regard qui accompagne cette vision. Le document de brevet EP1882444 décrit une méthode et un dispositif pour mesurer les caractéristiques visuelles d'un oeil selon différentes directions du regard, dans lequel un aberromètre est placé sur un support mobile en rotation de manière à incliner l'axe de mesure pour l'aligner suivant une direction du regard abaissé. Cependant, si l'on souhaite utiliser un appareil commercial actuel dans une direction naturelle d'abaissement ou d'élévation du regard, une difficulté technique apparaît (impossibilité dans certains cas) pour positionner la voie de mesure dans l'axe de regard naturel du sujet. On se trouve en effet confronté à des collisions entre la tête du sujet et l'appareil de mesure, les éléments mécaniques des systèmes existants, en particulier les platines en translation pour le centrage, étant conçus pour fonctionner sur un plan horizontal.

Il n'existe pas actuellement d'appareil d'optométrie, de type autoréfracteur ou aberromètre, permettant d'étudier le phénomène de réfraction différenciée en vision de loin et en vision de près suivant l'abaissement naturel du regard.

### OBJET DE L'INVENTION

On cherche à améliorer la précision des mesures ophtalmologiques différenciées en fonction de la distance de vision du sujet et de l'abaissement du regard, pour améliorer la compensation différenciée apportée selon les conditions de vision d'un porteur de lentilles de lunettes multifocales ou progressives. En particulier, on cherche à effectuer des mesures d'astigmatisme et/ou des mesures d'aberrations d'ordre supérieur différenciées en VL/VP.

Un des buts de l'invention est de fournir un dispositif et une méthode d'optométrie pour effectuer une mesure de réfraction oculaire différenciée VL/VP d'un sujet en conditions naturelles d'abaissement du regard.

L'invention vise à proposer un dispositif d'optométrie pour mesurer au moins un paramètre de vision pour différentes distances et pour différentes directions de vision déterminées. En particulier, l'invention vise à proposer un dispositif d'optométrie permettant d'effectuer des mesures selon des distances et des directions qui suivent l'abaissement naturel de regard du sujet.

L'invention vise aussi à réaliser une mesure précise de la variation des paramètres de vision en fonction de l'abaissement du regard avec des moyens de mesures objectifs, c'est-à-dire sans nécessiter de moyens de mesures subjectifs longs et complexes à mettre en oeuvre (par exemple via une lunette d'essais ou un réfracteur).

Afin de remédier à l'inconvénient précité de l'état de la technique, la présente invention propose un dispositif selon la revendication 1.

D'autres caractéristiques non limitatives et avantageuses du dispositif de détermination d'au moins un paramètre de réfraction oculaire objective d'un sujet en fonction d'une pluralité de directions de regard du sujet conforme à l'invention sont les suivantes :
- ledit premier angle d'abaissement du regard est un angle nul par rapport à l'horizontale et ladite première valeur de proximité est comprise entre 0 et 10 dioptries ;
- lesdits moyens opto-mécaniques d'alignement comprennent au moins une lame semi-réfléchissante et au moins un obturateur, ladite au moins une lame semi-réfléchissante étant disposée sur ledit au moins un axe optique de mesure de manière à définir un premier axe secondaire de mesure et au moins un autre axe secondaire de mesure, ledit au moins un obturateur commutant de manière à obturer ledit premier ou ledit au moins un autre axe secondaire de mesure, ledit premier axe secondaire de mesure étant destiné à être aligné sur un axe oculaire orienté suivant un premier angle d'abaissement du regard et ledit au moins un autre axe secondaire de mesure étant destiné à être aligné sur un axe oculaire orienté suivant un autre angle d'abaissement du regard ;
- lesdits moyens opto-mécaniques d'alignement comprennent des moyens de déplacement desdits moyens de mesure ophtalmologique et des moyens de guidage mécanique des moyens de déplacement suivant une trajectoire prédéterminée en fonction de l'angle d'abaissement du regard et de l'écartement pupillaire d'un sujet de référence.

Avantageusement, lesdits moyens de guidage mécanique comprennent un premier rail de guidage en forme de portion d'une première sphère et/ou un deuxième rail de guidage en forme de portion d'une deuxième sphère, la première sphère étant centrée sur le centre de rotation optique de l'oeil mesuré droit et/ou respectivement la deuxième sphère étant centrée sur le centre de rotation optique de l'oeil mesuré gauche du sujet.

Avantageusement, le dispositif de l'invention comprend en outre des moyens de détection de la direction et de la position du regard dudit sujet dans ladite première position de mesure P0 et dans ladite au moins une autre position de mesure PN, et des moyens d'asservissement pour asservir ledit premier alignement optique dudit au moins un axe optique de mesure sur l'axe oculaire droit, et/ou respectivement gauche dans ladite première position de mesure P0 et pour asservir ledit au moins un autre alignement optique dudit au moins un axe optique de mesure sur l'axe oculaire droit, et/ou respectivement gauche, dans ladite au moins une autre position de mesure PN.

Avantageusement encore, lesdits moyens opto-mécaniques d'alignement comprennent des moyens d'ajustement de la distance inter-pupillaire ou de la demi-distance inter-pupillaire gauche et de la demi-distance inter-pupillaire droite.

Selon certains aspects particuliers :
- l'angle d'abaissement du regard par rapport à l'horizontale est compris entre -30 degrés et +70 degrés ;
- la valeur de proximité desdits moyens de stimulation visuelle est comprise entre -3 et +10 dioptries en proximité ;
- le dispositif comprend une première position prédéterminée pour ledit au moins un premier angle d'abaissement du regard et une deuxième position prédéterminée pour ledit au moins un autre angle d'abaissement du regard ;
- le dispositif de mesure comprend des premiers moyens de mesure ophtalmologique de la réfraction oculaire de l'oeil droit, des premiers moyens de stimulation visuelle de l'oeil droit, des deuxièmes moyens de mesure ophtalmologique de la réfraction oculaire objective de l'oeil gauche, et des deuxièmes moyens de stimulation visuelle de l'oeil gauche, lesdits premiers et deuxièmes moyens de stimulation, ayant une même valeur de proximité pour un même angle d'abaissement du regard ;
- le dispositif comprend au moins un système optique apte à compenser au moins l'un des défauts de réfraction suivant : un défaut de sphère, de cylindre et d'axe et/ou d'aberrations d'ordre supérieur pour chaque oeil mesuré.

L'invention propose également un procédé selon la revendication 12.

Selon des aspects particuliers et avantageux du procédé selon l'invention :
- la première mesure ophtalmologique de réfraction oculaire objective pour ledit premier couple angle d'abaissement du regard et proximité est effectuée à un premier instant t0, et ladite au moins une autre mesure ophtalmologique de réfraction oculaire objective pour ledit autre couple angle d'abaissement du regard et proximité est effectuée à un autre instant t0+T, les instants t0 et t0+T étant prédéterminés ;
- au moins un paramètre de réfraction oculaire objective mesuré est sélectionné de manière non limitative parmi : sphère, cylindre, axe, aberrations d'ordre supérieur, kératométrie et topographie cornéenne ou un différentiel d'un de ces paramètres entre deux angles d'abaissement du regard ;
- la mesure différentielle objective est utilisée comme donnée d'entrée dans la conception de fabrication d'une lentille correctrice de lunettes progressive ou multifocale.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente schématiquement un dispositif monoculaire selon un premier mode de réalisation de l'invention ;
- la figure 2 représente en vue de dessus un dispositif binoculaire d'optométrie différencié VL/VP selon un deuxième mode de réalisation de l'invention ;
- les figures 3A-3E représentent schématiquement un accessoire pour dispositif monoculaire ou binoculaire selon un mode de réalisation préféré de l'invention ;
- la figure 4 représente schématiquement un dispositif binoculaire selon un troisième mode de réalisation de l'invention ;
- la figure 5 représente schématiquement un dispositif monoculaire ou binoculaire selon un quatrième mode de réalisation de l'invention ;
- la figure 6 représente schématiquement un dispositif monoculaire ou binoculaire selon un mode de réalisation de l'invention ;
- la figure 7 représente schématiquement une vue en perspective mettant en évidence les axes oculaires droit et gauche en fonction de l'abaissement du regard ainsi qu'un système de guidage pour suivre la direction du regard ;
- la figure 8 représente schématiquement une vue de côté (Fig. 8A) et une vue de face (Fig. 8B) des axes oculaires droit et gauche en fonction de l'abaissement du regard ainsi qu'un système de guidage pour suivre la direction du regard ;
- la figure 9 représente schématiquement une vue en perspective d'un dispositif binoculaire selon un mode de réalisation de l'invention ;
- la figure 10A-10B représente schématiquement une vue de côté (Fig. 10A) et une vue de face (Fig. 10B) du dispositif de la figure 9 pour une mesure avec un abaissement de regard nul ;
- la figure 11 représente schématiquement une vue de dessus du dispositif de la figure 9 pour une mesure avec un abaissement de regard nul ;
- la figure 12 représente schématiquement une vue de côté (Fig. 12A) et une vue de face (Fig. 12B) du dispositif de la figure 9 pour une mesure avec un abaissement de regard positif ;
- la figure 13 représente schématiquement une vue de dessus du dispositif de la figure 9 pour une mesure avec un abaissement de regard positif.

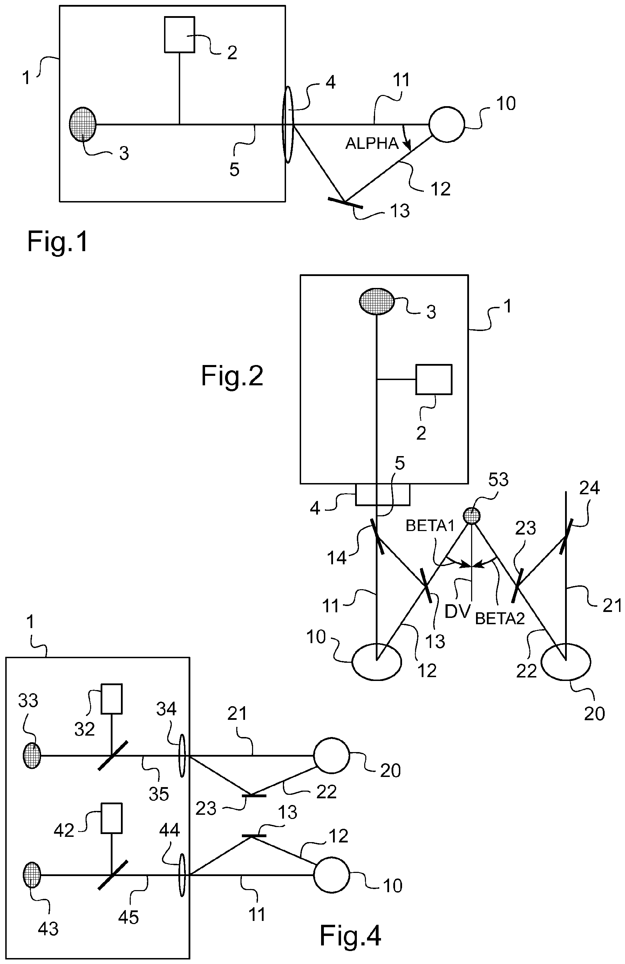

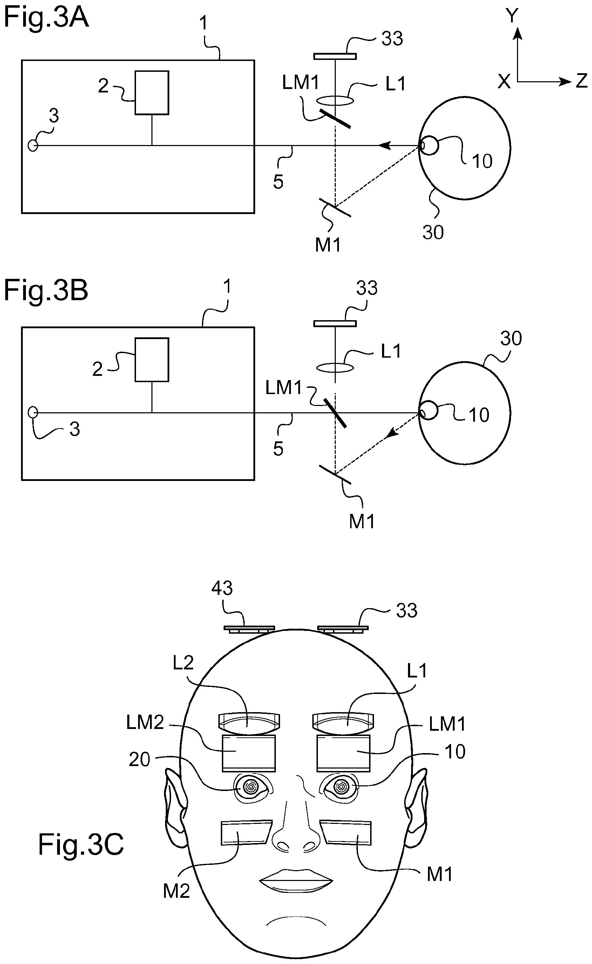

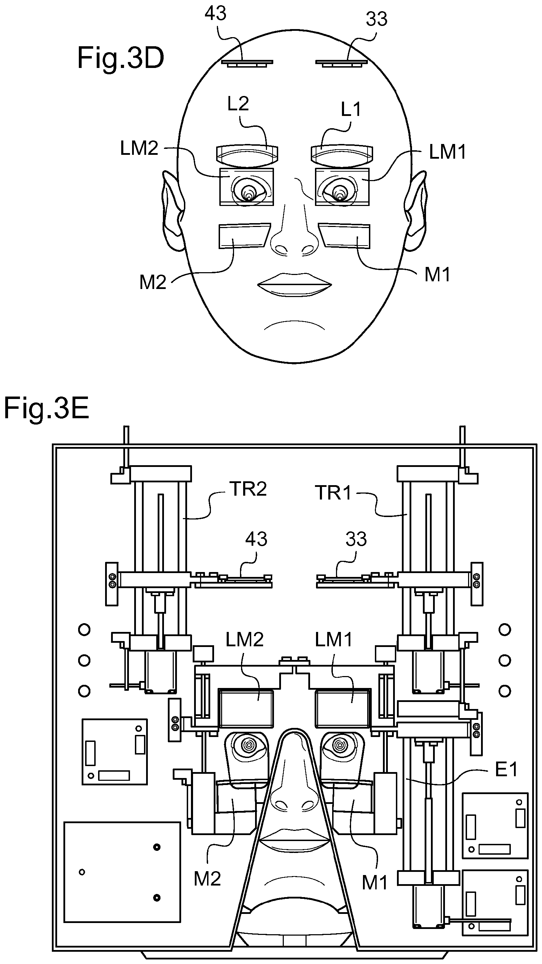

On définit un plan médian ou sagittal PSAG de la tête du sujet 30 perpendiculaire au plan de la figure 2 et passant par le milieu des deux yeux. Dans la description qui suit, on considère, comme illustré sur les figures 3 à 13 que le porteur est dans une configuration assise ou debout qui est telle que sa tête 30 est droite, c'est-à-dire que le plan de Francfort relatif à la tête du sujet est sensiblement horizontal. En anatomie, le plan de Francfort est le plan de repère qui permet l'étude du crâne. Autrement appelé plan de Virchow, il passe antérieurement par le plancher de l'orbite et postérieurement au dessus du méat acoustique externe. On dit également que le porteur est dans une position orthostatique, position dans laquelle il réalise le minimum d'efforts.

On définit l'axe de regard ou droite de visée DV du porteur située dans le plan sagittal du porteur. Dans le cas où le porteur regarde l'horizon droit devant lui à l'infini, la droite de visée est une droite horizontale DVI correspondant à l'axe de regard primaire. L'axe de regard du porteur est initialement horizontal. Au cours de la mesure différenciée décrite plus loin, le porteur est amené à abaisser ou à élever son regard. Dans le cas où le porteur abaisse le regard, la droite de visée DV est une droite située dans le plan sagittal et inclinée par rapport à une ligne horizontale. On définit un angle ALPHA comme angle d'abaissement du regard par rapport à l'horizontale. On définit l'axe oculaire droit comme étant l'axe passant par l'objet fixé par le porteur et le centre de la pupille de sortie (ie l'image de la pupille réelle par la cornée) de l'oeil droit. D'autres définitions sont possibles, par exemple, on peut prendre l'axe oculaire droit comme étant une droite passant par le centre de rotation de l'oeil droit et par le centre de la pupille de l'oeil droit ou encore l'axe reliant l'objet fixé à son image correspondant sur la rétine. Toutes ces définitions donnent approximativement le même axe. De même, on définit l'axe oculaire gauche comme étant l'axe passant par l'objet fixé par le porteur et le centre de la pupille de sortie de l'oeil gauche. L'angle ALPHA d'abaissement du regard est aussi défini comme étant l'angle formé dans le plan sagittal entre une droite horizontale (par exemple la droite DVI) et une projection de l'axe oculaire droit ou gauche dans le plan sagittal. L'angle ALPHA d'abaissement du regard peut prendre des valeurs positives (cas où le porteur abaisse effectivement le regard par exemple en position de lecture) ou des valeurs négatives (cas où le porteur élève le regard au dessus d'une ligne horizontale).

On définit aussi l'angle BETA de convergence du regard. Plus précisément, on définit l'angle BETA1 de convergence gauche formé entre la droite de visée DV et l'axe oculaire gauche du sujet. De même, on définit l'angle BETA2 de convergence droite formé entre la droite de visée DV et l'axe oculaire droit du sujet. Lorsque le sujet observe une cible située dans le plan sagittal, et que les demi-écarts pupillaires sont égaux, l'angle BETA1 est égal à -BETA2

La position dite « vision de loin » correspond à la vision d'un objet situé à l'infini face au porteur, la droite de visée étant horizontale. L'image de l'objet étant à l'infini, l'angle de convergence idéal des deux yeux est théoriquement nul. La vision de loin est donc associée à des paramètres de proximité nulle (0 dioptries) et d'angle d'abaissement du regard nul (ALPHA = 0). Il résulte de la proximité que l'angle de convergence effectif est généralement nul en vision de loin (BETA1= BETA2= 0). Néanmoins, si le sujet présente un défaut de convergence (convergence effective non nulle), il serait possible de le compenser si cela était jugé nécessaire, en utilisant des moyens appropriés tels qu'un prisme de compensation par exemple. La position dite vision de près correspond à la vision de l'image d'un objet située à une distance proche (de 20 à 40 cm par exemple) face au sujet, la droite de visée étant abaissée. En vision de près, les deux yeux convergent vers l'image de l'objet. La vision de près est donc associée à des paramètres de proximité non nulle (2 à 10 dioptries) et d'angle d'abaissement du regard non nul (ALPHA compris entre 20 et 70 degrés). Il résulte de la proximité que l'angle de convergence est généralement non nul en vision de près (BETA1= - BETA2 compris entre 3 degrés et 20 degrés). De même qu'en vision de loin, si le sujet présente un défaut de convergence (BETA1 et BETA2 effectifs différents en valeur absolue), il serait possible de le compenser si cela était jugé nécessaire par des moyens appropriés connus de l'homme du métier.

Ces positions de vision de loin et de vision de près correspondent à la vision naturelle d'un porteur de lentilles correctrices de lunettes multifocale ou progressive, qui modifie uniquement l'abaissement de son regard pour passer d'une vision de loin, par exemple pour la conduite automobile, à une vision de près par exemple pour la lecture d'un document papier. Une position de vision intermédiaire (VI) en termes de proximité (1D) et d'angle d'abaissement du regard (10 à 30°) correspond par exemple à la distance confortable pour la lecture sur un écran d'ordinateur situé à 1m.

La compensation optimale d'une lentille correctrice multifocale ou progressive varie non seulement en fonction de la proximité d'une cible mais varie conjointement en fonction de l'abaissement du regard. Des études de suivi de la cinématique des yeux d'un sujet en fonction de l'abaissement du regard ont permis d'analyser le mouvement des yeux lorsqu'un sujet passe d'une position naturelle en vision de loin, avec l'axe du regard horizontal, à une position en vision de près, avec l'axe du regard abaissé, par exemple pour la lecture d'un document papier. On observe non seulement une convergence des deux yeux, qui se traduit par une modification de la distance inter-pupillaire, mais aussi de façon non limitative une rotation de chaque oeil autour son axe oculaire, une augmentation de la pression de la paupière inférieure sur la cornée, le décentrement du cristallin avec l'accommodation. Il en découle que l'orientation de l'axe physiologique d'astigmatisme d'un oeil ainsi que la valeur de l'astigmatisme varie en passant d'une position naturelle de vision de loin et à la vision de près. Or, cette variation de l'astigmatisme entre la vision de près et la vision de loin n'est généralement pas prise en compte pour paramétrer une lentille correctrice multi-focale ou une lentille correctrice progressive. Plus généralement, il est souhaitable de mesurer précisément les paramètres de compensation oculaire (sphère, cylindre, axe, aberrations d'ordres supérieurs, kératométrie, topographie cornéenne...) en fonction de la proximité de la cible et en fonction de l'abaissement du regard pour compenser la vision en fonction de la position naturelle de l'oeil.

Nous allons maintenant détailler différents modes de réalisation du dispositif de l'invention qui permettent de contrôler les paramètres de mesure pour passer d'une mesure en vision de loin à une mesure en vision à une distance plus proche (entre autre VP).

### Dispositif

Sur la figure 1, on a représenté en vue de côté un dispositif monoculaire d'optométrie différencié en vision de loin et en vision de près selon un premier mode de réalisation de l'invention. Le dispositif d'optométrie 1 comprend un système de mesure par réfraction oculaire 2 ; une cible 3 de proximité variable pour stimuler l'accommodation du sujet en vision de loin et en vision de près. Le système de mesure 2 émet un faisceau lumineux suivant un axe optique 5 en direction de l'oeil 10 du sujet à mesurer et collecte le faisceau lumineux provenant de la réfraction sur l'oeil suivant ce même axe optique 5. La cible 3 émet un faisceau lumineux suivant un axe colinéaire avec l'axe optique de mesure 5. Le dispositif de mesure différenciée VL/VP comporte en outre un système optique (non représenté) sur le trajet optique de la cible 3 pour modifier la valeur de proximité de la cible par rapport à l'oeil d'un sujet. A cet effet, on utilise avantageusement un système optique de puissance variable pour modifier la proximité de la cible. Le système de puissance variable peut aussi être utilisé afin de compenser l'amétropie en vision de loin lors d'une mesure en vision de près regard abaissé (compensation de la sphère en vision de loin et ajout de l'addition lors de la mesure en regard abaissé), ce qui permet alors au sujet de voir net la cible en regard abaissé. De manière alternative, on peut utiliser un système optique escamotable, le système optique étant retiré du trajet optique dans une première position de mesure (en vision de loin par exemple) et inséré sur le trajet optique dans une deuxième position de mesure (en vision de près par exemple). On peut alternativement utiliser un système dit Badal qui utilise un déplacement de la cible associé à un système optique dont le foyer est confondu avec la position de la pupille de l'oeil du sujet. Ce système permet notamment de conserver une taille apparente de l'image de la cible constante pour toutes les valeurs de proximité.

Le dispositif d'optométrie par réfraction oculaire comporte aussi une pupille d'entrée-sortie 4. Le dispositif de mesure différencié comporte en outre un système optique à miroir 13 pour modifier la direction de l'axe optique de mesure de manière à modifier l'angle d'abaissement du regard lorsque la proximité de la cible est modifiée. Un obturateur ou un miroir commutable permet d'aligner l'axe optique de mesure 5 vers une première ou vers une autre direction, une seule direction de mesure étant active à la fois. Sur la figure 1, on a aussi représenté l'oeil 10 d'un sujet dont on cherche à mesurer les paramètres de vision différenciée. Dans une première position P0 de l'appareil de mesure, en vision de loin, le sujet regarde droit devant lui. La première valeur de proximité D0 de la cible est fixée par exemple à 0 dioptrie. L'axe oculaire 11 de l'oeil 10 est alors horizontal. On aligne dans cette première position P0, l'axe optique 5 de l'appareil de mesure sur l'axe oculaire 11 en vision de loin. On utilise par exemple une caméra pour visualiser la pupille de l'oeil et aligner l'axe optique de l'appareil de mesure sur l'axe oculaire concerné. Dans la première position de mesure P0 en vision de loin, on peut ainsi mesurer la réfraction oculaire sur l'oeil 10 pour la première valeur de proximité D0 de la cible et pour l'angle d'abaissement du regard nul. Dans une deuxième position P1 de l'appareil de mesure, en vision de près, on ajuste la valeur de proximité D1 de la cible à une deuxième valeur de proximité, par exemple de 2.5 dioptries, de manière à stimuler l'accommodation de l'oeil en vision de près. Simultanément, on commute l'obturateur situé à l'extérieur de l'appareil de mesure 1 pour stimuler l'abaissement et éventuellement la convergence du regard, l'axe oculaire en vision de près 12 étant dans cette position aligné sur l'axe optique de mesure et de stimulation, par l'intermédiaire du système optique à miroir 13. Le système optique à miroirs 13 et l'obturateur permettent de commuter l'axe optique de mesure entre une première position où il est aligné sur l'axe 11 horizontal en vision de loin et une deuxième position où il est aligné sur l'axe 12 abaissé en vision de près. On peut utiliser tout système optique adapté pour commuter l'axe optique de mesure entre deux positions. Dans la deuxième position P1, le sujet regarde la cible 3 dont l'image est à une deuxième valeur de proximité D1 et suivant une direction d'abaissement du regard inclinée d'un angle ALPHA prédéterminé, et éventuellement avec un angle BETA de convergence du regard. De manière analogue, on aligne dans cette deuxième position P1, l'axe optique 5 de l'appareil de mesure sur l'axe oculaire 12 en vision de près. Dans la deuxième position de mesure P1 en vision de près, on peut ainsi mesurer la réfraction oculaire sur l'oeil 10 pour une deuxième valeur de proximité de la cible et pour un angle d'abaissement du regard compris préférentiellement entre 20 et 36 degrés. On obtient ainsi une première mesure de réfraction oculaire pour un premier ensemble de valeurs de proximité et d'angle d'abaissement du regard (par exemple D0= 0 dioptrie, ALPHA = 0 degré) et une deuxième mesure de réfraction oculaire pour un deuxième ensemble de valeurs de proximité et d'angle d'abaissement du regard (par exemple D1= 2.5 dioptries, ALPHA = 36 degrés). A partir de ces mesures, un calculateur en déduit une mesure de réfraction oculaire différenciée VL/VP en fonction de l'abaissement du regard sur un oeil.

Sur la figure 2, on a représenté en vue de dessus un dispositif binoculaire d'optométrie différencié en vision de loin et en vision de près selon un deuxième mode de réalisation de l'invention. Le dispositif d'optométrie 1 comprend un système de mesure 2 monoculaire par réfraction ayant une pupille 4 d'entrée-sortie et une cible 3 pour stimuler l'accommodation en vision monoculaire. Le système de mesure 2 émet un faisceau lumineux suivant un axe optique 5 en direction d'un oeil du sujet à mesurer et collecte le faisceau lumineux provenant de la réfraction sur l'oeil suivant ce même axe optique 5. Le système de mesure 2 est monté sur un charriot de déplacement latéral monté par exemple sur un rail de translation (non représenté sur la figure 2) qui permet d'aligner le système de mesure soit sur l'axe oculaire de l'oeil gauche 10 (comme sur la figure 2) soit sur l'axe oculaire de l'oeil droit 20. Ce moyen de déplacement permet d'effectuer séquentiellement une mesure monoculaire de réfraction oculaire de chacun des deux yeux pour en déduire une mesure binoculaire. La direction de l'axe optique de mesure 5 reste inchangée quel que soit l'oeil mesuré, et quelle que soit la direction de l'axe de visée du regard.

Un accessoire de mesure pluri-directionnel est disposée à l'extérieur du système de mesure 2. L'accessoire de mesure pluri-directionnel comprend une cible secondaire 53 et un système optique à miroirs et/ou à lames semi-transparentes. Avantageusement, la cible secondaire 53 est disposée dans le plan sagittal du sujet et la cible 53 émet un faisceau lumineux identique en direction de l'oeil gauche 10 et de l'oeil droit 20 du sujet. La cible secondaire 53 présente une proximité variable pour stimuler l'accommodation et la convergence des deux yeux simultanément. Le dispositif de la figure 2 comporte en outre un premier système optique comprenant des lames semi-transparentes 13 et 14 et un obturateur commutable (non représenté sur la figure 2) entre une première position où la lame 14 est à l'écart du chemin optique de l'axe oculaire gauche en VL 11 et une deuxième position où la lame 14 est interposée sur le chemin optique de l'axe oculaire gauche en VP 12. Symétriquement, un deuxième système optique comprenant des lames semi-transparentes 23 et 24 et un obturateur commutable - non représenté- entre une première position où il obture le chemin optique de l'axe oculaire droit en VP 22 et une deuxième position où il obture le chemin optique de l'axe oculaire droit en VL 21. Le système optique formé des lames semi-transparentes 13, 14, 23, 24 et des obturateurs commutables permet de modifier la direction de l'axe optique de stimulation et de mesure de manière à modifier simultanément l'angle d'abaissement du regard et l'angle de convergence du regard lorsque la proximité de la cible est modifiée.

Dans une première position de mesure P0 de réfraction oculaire de l'oeil gauche en vision de loin, le système de mesure 1 de la Fig. 2 est positionné de manière à ce que l'axe optique de mesure 5 soit aligné sur l'axe oculaire de l'oeil gauche en vision de loin 11, l'obturateur étant inséré sur le chemin optique de l'axe 12. La proximité de la cible 3 est fixée à l'infini et la cible secondaire 53 est éteinte. La lame semi-réfléchissante 13 est disposée sur le trajet optique du faisceau de la cible 3 sans défléchir le faisceau de la cible 3 de manière à ce que le faisceau de la cible soit superposé avec l'axe oculaire 11 de l'oeil gauche en VL. Le deuxième système optique comprenant deux lames semi-réfléchissantes 23, 24 et un obturateur sur le chemin optique de l'axe oculaire droit en VP 22 est disposé symétriquement sur l'axe oculaire 21 de l'oeil droit 20 de manière à stimuler la vision de loin des deux yeux. Comme décrit en lien avec la figure 1, dans la première position de mesure P0 en vision de loin, on effectue une première mesure de la réfraction oculaire sur l'oeil gauche 10 pour la première valeur de proximité de la cible 3 et pour un premier angle d'abaissement du regard nul. Sans changer la proximité de la cible 3 ni l'angle d'abaissement du regard, le dispositif de mesure 1 est translaté latéralement de manière à aligner l'axe optique de mesure 5 sur l'axe oculaire de l'oeil droit en VL 21. De même, on effectue dans cette position une première mesure de la réfraction oculaire sur l'oeil droit 20 pour la première valeur de proximité de la cible 3 et pour un premier angle d'abaissement du regard nul. On obtient ainsi une première mesure binoculaire de la réfraction oculaire pour la première valeur de proximité D0, le premier angle d'abaissement du regard nul.

Dans une deuxième position de mesure P1, la cible 3 est éteinte et la cible secondaire 53 est allumée. La proximité de la cible 53 est amenée à une deuxième valeur de proximité D1. Conjointement, les deux obturateurs commutent pour libérer le chemin optique des axes oculaires droit 22 et gauche 12 en VP et obturer le chemin optique des axes oculaires droit 21 et gauche 11 en VL. Supposons que le système de mesure 2 soit de nouveau positionné face à l'oeil gauche 10. Les obturateurs empêchent le sujet de regarder droit devant lui (en VL) et stimulent le regard vers une position en vision de près. Le faisceau optique de mesure se superpose alors au faisceau cible et avec l'axe oculaire gauche 12 en VP. La cible 53 et les lames semi-transparentes 13, 23 sont disposées de manière à induire non seulement une convergence des deux yeux mais aussi un abaissement du regard d'un angle ALPHA, comme représenté sur la figure 1. Dans la deuxième position de mesure P1, la cible secondaire 53 induit, en projection dans le plan de la figure 2, un angle de convergence BETA1 de l'axe oculaire gauche 12 en VP et un angle de convergence BETA2 de l'axe oculaire droit 22 en VP. Conjointement, la cible secondaire 53 induit un angle d'abaissement du regard ALPHA des axes oculaires gauche 12 et droit 22 en VP. Dans la deuxième position de mesure P1 en vision de près, on effectue une deuxième mesure de la réfraction oculaire sur l'oeil gauche 10 pour la deuxième valeur de proximité D1 de la cible, pour un angle d'abaissement du regard ALPHA préférentiellement compris entre 20 et 40 degrés. De manière analogue, sans changer la proximité de la cible 53 ni l'angle d'abaissement du regard, le dispositif de mesure 2 est translaté de manière à aligner l'axe optique de mesure 5 sur l'axe oculaire de l'oeil droit en VP 22. De même, on effectue dans cette position une deuxième mesure de la réfraction oculaire sur l'oeil droit 20 pour la deuxième valeur de proximité D1 de la cible 53, pour le même angle d'abaissement du regard ALPHA et pour un angle de convergence BETA2 égal à -BETA1 (aux défauts de convergence près). On obtient ainsi une deuxième mesure binoculaire de la réfraction oculaire pour la deuxième valeur de proximité D1 et le deuxième angle d'abaissement du regard ALPHA.

Sur les figures 3A à 3E on a représenté un accessoire pour dispositif monoculaire ou binoculaire selon un mode de réalisation préféré de l'invention. Les figures 3A et 3B représentent le système dans son ensemble et les figures 3C et 3D représentent de manière détaillée les éléments placés entre le sujet et le système de mesure de réfraction 1. L'appareil de mesure comporte un système de réfraction automatique 1 capable de mesurer les valeurs optiques de sphère, cylindre et/ou axe (ou S, C, A) d'un oeil dont l'axe visuel est aligné avec l'axe de mesure 5. Ce système de mesure de réfraction automatique peut être par exemple un appareil de type autoréfracteur ou aberromètre. Le système de réfraction automatique comporte une source cible interne 3, qui peut être éteinte ou allumée lors de la mesure de réfraction et dont la proximité peut être réglée. L'axe de stimulus défini par cette cible est confondu avec l'axe de mesure 5.

Le système de mesure de réfraction automatique est mobile suivant les axes X,Y et Z de manière automatique ou manuelle. L'axe Z est utilisé pour réaliser une mise au point nette de l'image de la pupille et ainsi assurer une mesure de la réfraction et un bon alignement de l'axe de visée avec l'axe de mesure 5. L'axe X est utilisé pour aligner l'axe de visé et l'axe de mesure horizontalement, et pour passer d'un oeil droit à un oeil gauche. L'axe Y est utilisé pour aligner l'axe de visé et l'axe de mesure 5 verticalement.

La cible 3 est allumée et sert de cible de fixation pour la mesure dans une première position, et est éteinte sinon. Deux cibles complémentaires 33 et 43 sont utilisées et servent de cible de fixation lors de la mesure dans une deuxième position. Les cibles complémentaires 33 et 43 sont utilisées préférentiellement en simultané, afin de favoriser la convergence des deux yeux. Ces deux cibles 33, 43 sont montées sur des systèmes de translation TR1 et TR2 motorisés (voir fig. 3E en vue de face), pour permettre de déplacer les cibles suivant l'axe Y et ainsi faire varier la proximité. Pour optimiser la compensation de l'amétropie, on peut aussi ternir compte du cylindre VL. Les deux cibles 33, 43 quand elles sont utilisées simultanément, ont des caractéristiques communes suffisantes pour stimuler la fusion de manière à représenter une cible identique pour l'oeil droit et gauche. Les cibles 33, 43 possèdent de plus une mobilité suivant l'axe X, cette mobilité étant utilisée afin de compenser un défaut résiduel de convergence. Les cibles 33, 43 prennent la forme préférentielle d'écran graphique miniature, type écran OLED, ou d'une matrice de Leds, ou de sources mécaniquement mobiles en X.

Deux lentilles L1 et L2 dont les foyers correspondent à la position des pupilles des yeux droit/gauches sont placées entre les yeux du sujet et les cibles 33 et 43. Une lame chaude (réfléchissant l'infrarouge mais transparente dans le visible) LM1 est placée entre le système de mesure de réfraction automatique 1 et les yeux du sujet. La lame LM1 est rendue escamotable par un mécanisme E1, entre deux positions : une position pour laquelle la lame LM1 est à 45° par rapport à l'axe de mesure 5, et une position où la lame LM1 est en retrait de l'axe de mesure 5. Deux miroirs M1 et M2, inclinés suivant les axes X et Z, sont placés en face de chaque oeil de manière à compléter le système.
Le fonctionnement du dispositif est le suivant :
Pour la première position P0, les cibles 33 et 43 sont éteintes et la cible 3 est allumée (voir la figure 3A en vue de côté et la figure 3C en vue de face). La proximité de la cible 3 est fixée à 0D, la lame LM1 est en retrait de l'axe de mesure 5, et le système de mesure 1 est déplacé en X, Y, Z de manière à ce que l'axe de mesure 5 passe par la pupille de l'oeil droit, qui fixe la cible 3. Ce déplacement est réalisé manuellement, en observant par exemple une image de l'oeil et en assurant que la pupille est bien centrée sur l'axe de mesure 5, ou de manière automatique par détection automatique de la pupille de l'oeil et asservissement automatique en position. Quand le réglage en X,Y,Z est réalisé, l'axe oculaire est alors horizontal et confondu avec l'axe de mesure 5. On mesure alors les paramètres optiques S, C, Axe pour l'oeil mesuré correspondant à cette première position P0. On réalise de la même manière la mesure sur le second oeil, et on mémorise la position X, Y, Z pour chacun des yeux.
Pour la seconde position P1, on dispose les lames LM1 et LM2 devant les pupilles du sujet par translation, on éteint la cible 3, on allume les cibles 33 et 43 (voir la figure 3B en vue de côté et la figure 3D en vue de face). Les miroirs M1 et M2 sont positionnés de manière à ce que l'axe du regard soit incliné d'environ 30 degrés par rapport à l'horizontale, et pour une convergence à une proximité d'environ 40cm et un écart pupillaire moyen de 65mm. Les cibles 33 et 43 sont translatées par les moyens T1 et T2 de manière à ce que l'image des cibles 33 et 43 par les lentilles L1 et L2 soit à une distance d'environ 40cm. Dans le cas d'un écart pupillaire valant 65mm, les cibles 33 et 43 ne sont pas translatées suivant X. Si l'écart pupillaire s'éloigne de cette valeur moyenne, ou si la proximité est modifiée, alors on règle les cibles 33 et 43 de manière à ce que les axes oculaires droit et gauche se croisent pour la valeur de proximité choisie.

Pratiquement, pour une proximité de 40cm et un écart pupillaire supérieure (respectivement inférieur) à 65mm, on rapproche (respectivement on éloigne) les deux cibles 33 et 43 suivant X. Pour un écart pupillaire de 65mm et une proximité augmentée (respectivement diminuée), on rapproche (respectivement on éloigne) les deux cibles 33 et 43 suivant X. En pratique, si on utilise des écrans miniatures pour les cibles 33 et 43, on décale horizontalement l'affichage des cibles sur chacun des écrans du nombre de pixels adaptés.

On repositionne alors le système de mesure en X, Y, Z de manière à ce que l'axe oculaire après réflexion sur le miroir M1 (respectivement M2) et la lame LM1 (respectivement LM2) soit confondu avec l'axe optique de mesure 5.

On réalise alors la mesure des paramètres S,C, axe pour cette seconde position P1, pour les deux yeux. En pratique, l'écart pupillaire est déterminé lors de la mesure suivant la première position en mémorisant les positions X pour chacun des yeux. Préférentiellement, la tête du sujet est maintenue à l'aide d'un support de tête en appui frontal et d'une mentonnière 9, seuls les yeux bougent entre les positions de mesures P0 et P1 (voir figure 3E).

On peut aussi utiliser la mobilité des cibles 33 et 43 suivant les axes de translation TR1 et TR2 pour modifier la proximité des cibles vues à travers les lentilles L1 et L2, de manière à optimiser la compensation de l'amétropie du sujet et permettre d'avoir une image nette des cibles en position P1.

Sur la figure 4, on a représenté en vue de dessus un dispositif binoculaire d'optométrie différencié en vision de loin et en vision de près selon un troisième mode de réalisation de l'invention. Le dispositif d'optométrie comprend un premier système de mesure 42 monoculaire par réfraction ayant une première pupille 44 d'entrée-sortie, une première cible 43 et un premier axe optique de mesure 45 destiné à être aligné sur l'axe oculaire de l'oeil gauche 10. Le dispositif d'optométrie comprend un deuxième système de mesure 32 monoculaire par réfraction ayant une deuxième pupille 34 d'entrée-sortie, une deuxième cible 33 et un deuxième axe optique de mesure 35 destiné à être aligné sur l'axe oculaire de l'oeil droit 20. Le dispositif comporte également des moyens pour ajuster le demi-écart pupillaire gauche et droit. Chaque système de mesure 32, 42 fonctionne comme le dispositif décrit en lien avec la figure 1. Avantageusement, le dispositif de la figure 4 permet d'effectuer simultanément les premières mesures de réfraction oculaire en VL sur l'oeil gauche et sur l'oeil droit, la valeur de proximité de la première et de la deuxième cible 33, 43 étant fixées à une première valeur D0, le premier axe optique de mesure 45 étant aligné sur l'axe oculaire gauche en VL 11 et le deuxième axe optique de mesure 35 étant aligné sur l'axe oculaire droit en VL 21. Le dispositif de la figure 4 permet aussi d'effectuer simultanément les deuxièmes mesures de réfraction oculaire en VP sur l'oeil gauche et sur l'oeil droit, la valeur de proximité de la première et de la deuxième cible 33, 43 étant fixées à une deuxième valeur D1, le premier axe optique de mesure 45 étant aligné sur l'axe oculaire gauche en VP 12 et le deuxième axe optique de mesure 35 étant aligné sur l'axe oculaire droit en VP 22. On obtient ainsi un dispositif de mesure binoculaire différencié VL/VP sans avoir besoin de déplacer un appareil monoculaire séquentiellement devant chaque oeil.

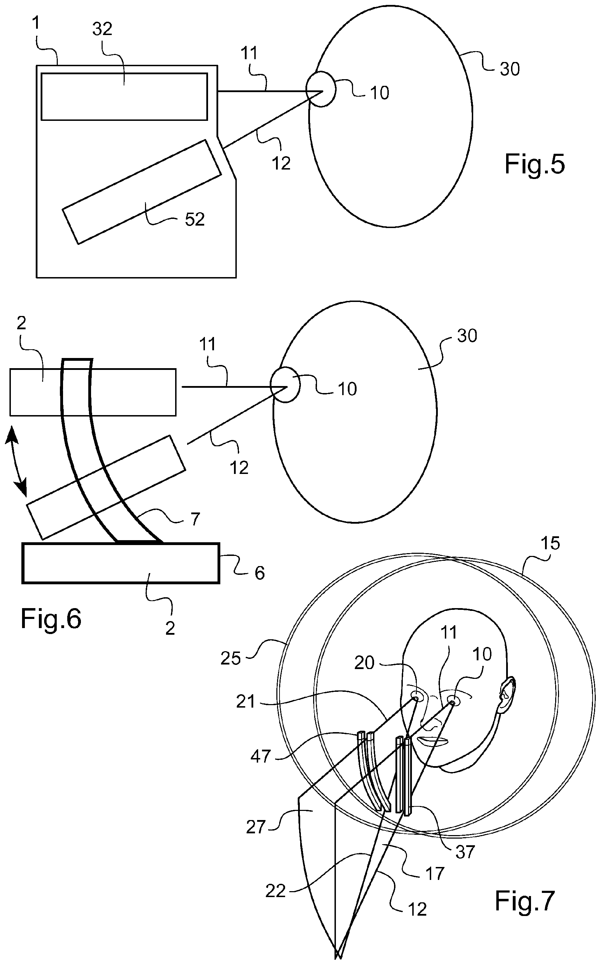

Sur la figure 5, on a représenté en vue de côté un dispositif d'optométrie différencié VL/VP selon un quatrième mode de réalisation. Le dispositif de mesure 32 comporte un appareil de mesure de la réfraction oculaire et une cible alignés sur un même axe optique, comme le dispositif 1 de la figure 1 par exemple. Le dispositif de mesure est mobile entre deux positions indiquées par les repères 32 et 52 sur la Fig. 5. La première position du système de mesure 32 correspond à une première mesure en VL pour une première valeur de proximité et pour un premier angle ALPHA1 d'abaissement du regard. La deuxième position du système de mesure 52 correspond à une deuxième mesure en VP pour une deuxième valeur de proximité D1 et pour un deuxième angle ALPHA d'abaissement du regard. Alternativement, l'appareil de mesure différencié comporte deux systèmes de mesure 32 et 52 dans deux positions prédéterminées. Le système représenté sur la figure 5 est soit un système monoculaire, soit un système binoculaire par déplacement latéral d'un système monoculaire, soit un système binoculaire comprenant un double système monoculaire, comme le dispositif de la figure 3. Le dispositif de la figure 5 permet une mesure différenciée entre deux positions prédéterminées, mais ne dispose pas de position de mesure intermédiaire.

Sur la figure 6, on a représenté en vue de côté un dispositif d'optométrie différencié VL/VP selon un autre mode de réalisation. Le dispositif de la figure 6 comporte un appareil de mesure de la réfraction oculaire 2 et une cible (non représentée) alignés sur un même axe optique, comme dans le dispositif 1 de la fig.1 par exemple. Le dispositif de mesure 2 est mobile le long d'un système de guidage 7 de forme inclinée entre une première et une deuxième position d'extrémité, la première position extrême de l'appareil de mesure 2 correspondant par exemple à la vision de loin lorsque l'axe de mesure est aligné sur l'axe oculaire 11 en vision de loin, et respectivement la deuxième position extrême de l'appareil de mesure 2 correspondant à la vision de près lorsque l'axe de mesure est aligné sur l'axe oculaire 12 en vision de près. Le dispositif de mesure de la figure 6 permet aussi d'effectuer des mesures de la réfraction oculaire dans au moins une position intermédiaire correspondant à une troisième valeur de proximité de la cible et à un troisième angle d'abaissement du regard. Le système de guidage 7 est fixé sur un socle 6 posé par exemple sur une table. Avantageusement, le système de guidage permet de maintenir l'alignement optique entre le système de mesure et l'oeil du sujet quel que soit l'angle d'abaissement du regard. Avantageusement encore, la forme du système de guidage 7 permet de suivre une courbe reproduisant le mouvement naturel d'abaissement et de convergence du regard d'un sujet de référence.

Nous allons maintenant détailler un mode de réalisation d'un dispositif de mesure de la réfraction oculaire différenciée en fonction de la proximité et de l'angle d'abaissement du regard en lien avec les figures 7 à 13.

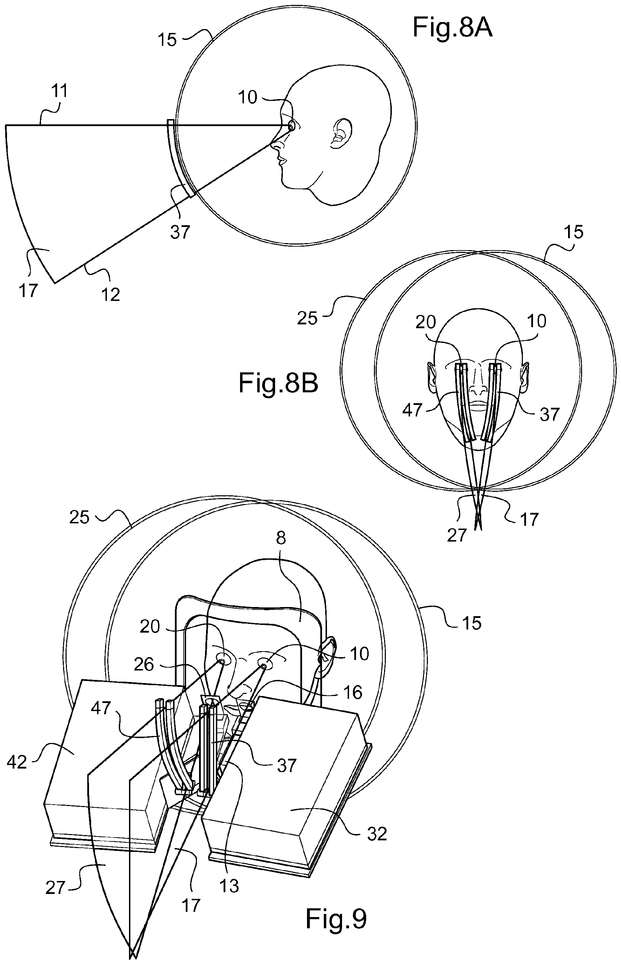

Avantageusement, le dispositif de mesure comporte un premier système de mesure de la réfraction oculaire gauche 42 avec une première cible 43 et un deuxième système de mesure de la réfraction oculaire droit 32 avec une deuxième cible 33 comme décrit en lien avec le mode de réalisation de la figure 4. Pour la clarté des figs. 7 et 8, ces éléments ne sont pas représentés mais fonctionnent comme décrit précédemment. Avantageusement, le dispositif de mesure comporte des moyens pour déplacer le premier et le deuxième système de mesure 42, 32 suivant une trajectoire type prédéfinie et permettant de toujours pointer le centre de rotation optique (CRO) de chaque oeil.

Sur la figure 7, on a représenté un système de guidage comprenant deux paires de rails de guidage 37 et 47 pour guider le premier système de mesure de la réfraction oculaire de l'oeil gauche 32 et respectivement le deuxième système de mesure de la réfraction oculaire de l'oeil droit 42. A cet effet, la trajectoire de l'appareil de mesure (32, respectivement 42) s'inscrit sur la surface d'une sphère (15, respectivement 25) dont le centre est le CRO de l'oeil gauche 10, respectivement de l'oeil droit 20. La trajectoire le long des rails de guidage 37, 47 permet de décrire un mouvement type entre la VL et la VP en tenant compte de l'angle d'abaissement du regard, de la convergence et/ou de la rotation de l'oeil.

Sur la Figure 8A, on a représenté une vue de côté d'un rail de guidage 37 situé sur une sphère 15 centrée sur le CRO de l'oeil gauche 10. Le rail de guidage impose une trajectoire circulaire entre une première position de mesure en VL, où l'axe de mesure est aligné sur l'axe oculaire 11 en VL et une deuxième position de mesure en VP, où l'axe de mesure est aligné sur l'axe oculaire 12 en VL. Une ou plusieurs positions de mesure intermédiaires sont possibles entre la première position de mesure en VL et la deuxième position de mesure en VP. L'abaissement du regard suit une surface 17 entre la première et la deuxième position de mesure. Sur la Figure 8B, on a représenté une vue de face des deux paires de rails de guidage 37 et 47. Le rail de guidage 37 est situé sur une sphère 15 centrée sur le CRO de l'oeil gauche 10 et le rail de guidage 47 est situé sur une sphère 25 centrée sur le CRO de l'oeil droit 20. Avantageusement, la première sphère et la deuxième sphère ont le même rayon. Comme représenté sur la figure 8B, les deux paires de rails de guidage 37, 47 sont placées respectivement en face de chaque oeil en VP centré sur le CRO. Les deux trajectoires 17 et 27 se décalent vers le nez pour forcer la convergence du regard lorsque l'angle d'abaissement du regard croît.

La figure 9 représente schématiquement une vue en perspective d'un dispositif binoculaire de mesure différenciée selon un mode de réalisation de l'invention. On utilise deux systèmes de mesure : un premier système de mesure de la réfraction oculaire 32 de l'oeil gauche 10 et un deuxième système de mesure de la réfraction oculaire 42 de l'oeil droit 20. Le premier système de mesure 32 est fixé sur un chariot se déplaçant le long du rail de guidage 37 et le deuxième système de mesure 42 est fixé sur un chariot se déplaçant le long du rail de guidage 47. Le premier et le deuxième système de mesure 32, 42 sont positionnés pour une mesure VL puis sont déplacés le long des rails 37 et 47 de manière à être abaissés et rapprochés pour une mesure VP.

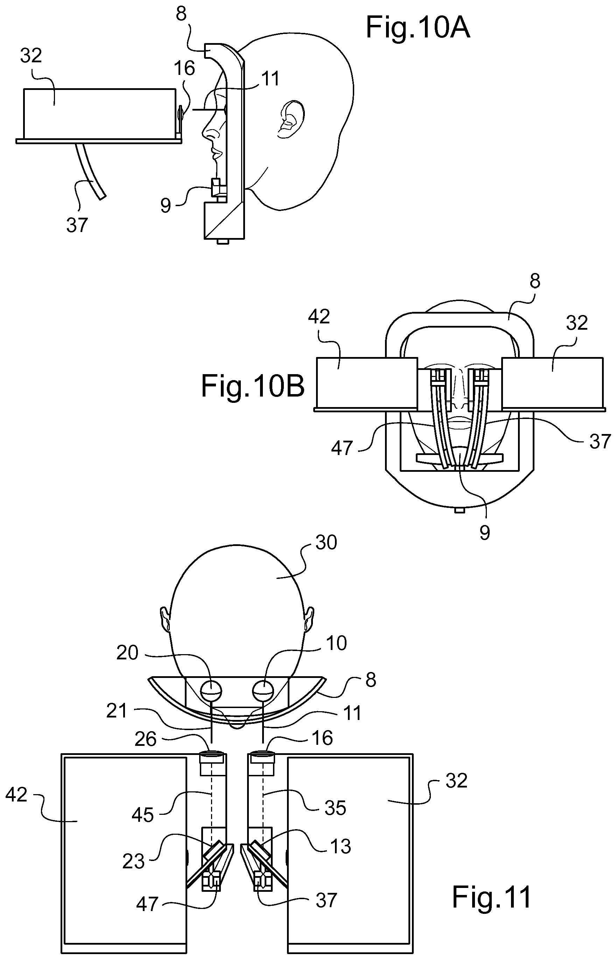

Les figures 10 et 11 représentent le dispositif de la figure 9 dans une première position de mesure en vision de loin VL. Plus précisément, la fig. 10A représente une vue de côté, la fig. 10B une vue de face et la fig. 11 une vue de dessus du dispositif de la figure 9 dans une première position de mesure en VL.

La tête de l'utilisateur 30 est maintenue dans une position fixe grâce à un support de tête 8 et à une mentonnière 9. Avantageusement, la tête 30 est maintenue dans une position où le plan de Francfort est horizontal. Alternativement, le support de tête permet de régler l'inclinaison de la tête dans une ou plusieurs autres positions de mesure. Le premier système de mesure 32 est fixé par l'intermédiaire d'un premier chariot au premier rail de guidage 37 et amené dans une première position. Un premier système optique comprenant un premier miroir de déflexion 13 et une première lentille 16 permet de défléchir l'axe optique de mesure du premier système de mesure 32. De même, le deuxième système de mesure 42 est fixé par l'intermédiaire d'un deuxième chariot au deuxième rail de guidage 47 et amené dans une première position. Un deuxième système optique comprenant un deuxième miroir de déflexion 23 et une deuxième lentille 26 permet de défléchir l'axe optique de mesure 45 du deuxième système de mesure 42. Ainsi, le premier et le deuxième système de mesure 32, 42 ne se gênent pas mutuellement quelles que soient leurs positions respectives le long des rails de guidage 37, 47. Dans la première position de mesure P0, l'axe optique 35 du premier système de mesure 32 est aligné sur l'axe oculaire gauche 11 en VL et l'axe optique 45 du deuxième système de mesure 42 est aligné sur l'axe oculaire droit 21 en VL. Dans cette première position, la proximité de la cible est ajustée à une première valeur de proximité D1, par exemple à l'infini. On peut alors effectuer une première mesure de la réfraction oculaire de l'oeil gauche 10 et de l'oeil droit 20 pour une première valeur de proximité D0 de la cible et pour une première valeur d'angle d'abaissement du regard, correspondant à la position de VL.

Avantageusement, le système optique 16, respectivement 26 est apte à compenser un défaut de sphère, de cylindre et d'axe, et/ou d'aberrations d'ordre supérieur pour chaque oeil mesuré 10, 20 de manière différenciée en fonction de l'angle d'abaissement du regard.

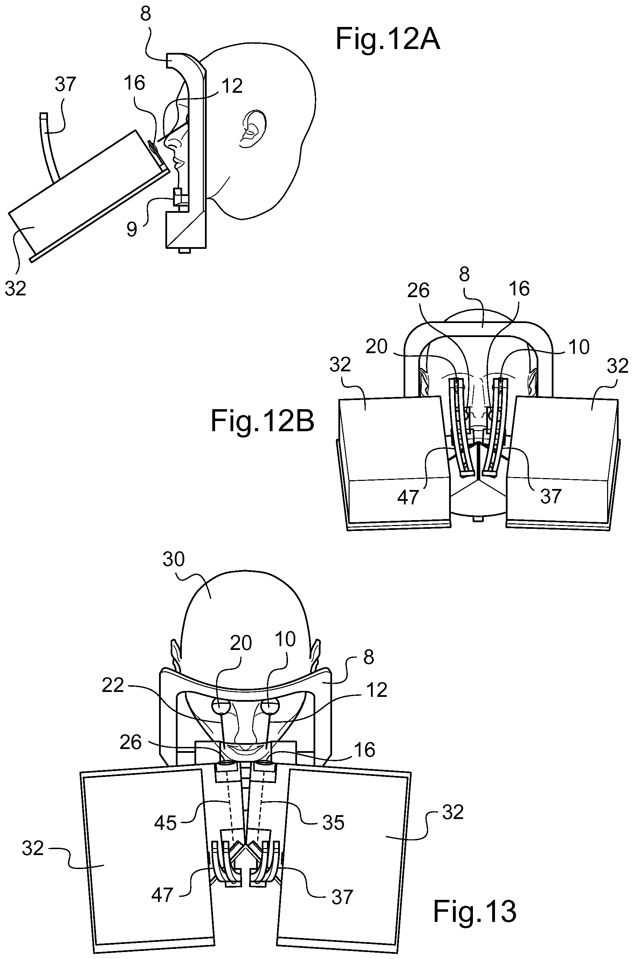

Les figures 12 et 13 représentent le dispositif de la figure 9 dans une deuxième position de mesure en vision de près (VP). Plus précisément, la fig. 12A représente une vue de côté, la fig. 12B une vue de face et la fig. 13 une vue de dessus du dispositif de la figure 9 dans une deuxième position de mesure en VP.

Le premier chariot supporte le premier système de mesure 32 et le premier système optique (premier miroir et première lentille 16) et le deuxième chariot supporte le deuxième système de mesure 42 et le deuxième système optique (deuxième miroir et deuxième lentille 26). Le premier chariot se déplace le long du rail 37 pour amener le système de mesure 32 dans une deuxième position de mesure en VP avec un abaissement du regard d'un angle ALPHA préférentiellement compris entre 20 et 40°. De préférence simultanément, le deuxième chariot se déplace le long du deuxième rail 47 pour amener le deuxième système de mesure 42 dans une deuxième position de mesure en VP avec un même abaissement du regard d'un même angle ALPHA. Dans cette deuxième position de mesure P1, l'axe optique 35 du premier système de mesure 32 est aligné sur l'axe oculaire gauche 12 en VP et l'axe optique 45 du deuxième système de mesure 42 est aligné sur l'axe oculaire droit 22 en VP. Dans cette deuxième position de mesure P1, la proximité des deux cibles est ajustée à une deuxième valeur de proximité D1, par exemple égale à 2.5 dioptries. On peut alors effectuer une deuxième mesure de la réfraction oculaire de l'oeil gauche 10 et de l'oeil droit 20 pour une deuxième valeur de proximité D1 de la cible et pour une deuxième valeur d'angle d'abaissement du regard, correspondant à la position de VP.

L'homme du métier comprendra que le dispositif de la figure 9 permet aisément d'effectuer au moins une mesure intermédiaire entre la première position de mesure P0 en VL et la deuxième position de mesure P1 en VP, pour un autre couple de valeur de proximité de la cible et d'angle d'abaissement du regard. Le dispositif permet avantageusement de suivre une trajectoire d'abaissement du regard et de convergence prédéfinie selon une loi type.

### Procédé

Le principe de la méthode est d'assurer en permanence un bon alignement des axes optiques de mesure avec les axes oculaires du sujet. Il convient également d'aligner les axes optiques des voies de stimulation visuelles avec les deux axes précédents.

Plus généralement, nous nous intéressons à l'ensemble des directions de regard possibles entre la direction correspondant à la vision de loin et la direction correspondant à la vision de près d'un sujet.

D'un point de vue général, on appelle la position de mesure regard droit devant ou position primaire du regard, avec un regard en vision de loin (VL), position initiale P0, et on appelle la mesure associée M0.

Pour la mise en place des conditions de mesures pour la position P0, on peut utiliser comme données d'entrée, les paramètres suivants :
- Sph VL
- Cyl VL
- Axe VL
- Addition
- ½ IPD G
- ½ IPD D

Sph VL correspond à une compensation de puissance ou sphère en vision de loin. Une compensation d'astigmatisme en vision de loin est définie par les paramètres Cyl VL (cylindre) et Axe VL (axe du cylindre). Ces valeurs correspondent, par exemple, soit à une ancienne prescription du sujet, soit au résultat d'une mesure de réfraction subjective réalisée préalablement par un optométriste, ophtalmologiste ou opticien suivant une méthode classique en VL. L'ensemble des paramètres d'entrée sont optionnels et permettent de bien paramétrer la cible 3 et éventuellement le module de mesure 2 dès le démarrage de la mesure du sujet. Ceci permet de prépositionner l'ensemble des éléments et donc de réaliser une mesure plus rapide. S'ils ne sont pas disponibles, ils sont alors intégralement mesurés par l'appareil.

Dans cette position initiale P0 en vision de loin (VL), il nous est possible d'obtenir les mesures suivantes :
- Sph VLm (sphère en vision de loin mesurée)
- Cyl VLm (cylindre en vision de loin mesuré)
- Axe VLm (axe du cylindre en vision de loin mesuré)
- ½ IPD Gm (option) (demi-écart pupillaire gauche mesuré)
- ½ IPD Dm (option) (demi-écart pupillaire droit mesuré)

Pour les autres directions de mesure, il nous est possible de contrôler les axes de mesure et de stimulation par rapport aux axes oculaires droit et/ou gauche de deux façons :
1) On utilise les contraintes liées aux lentilles correctrices progressives ou multifocales pour déterminer l'abaissement de regard du sujet pour passer de la position P0 à une position Pn qui correspond à une zone d'un verre progressif (donc à une puissance, donc à une proximité) ;
2) On effectue au préalable une mesure des paramètres naturels de posture d'un sujet pour effectuer une tâche déterminée (lecture en VP, consultation en VI, etc...).

Dans les deux cas on obtient, une proximité à appliquer à la cible 3, un angle d'abaissement de regard binoculaire correspondant, et un correctif à apporter à l'écart inter-pupillaire.

L'utilisation de ces trois paramètres permet de repositionner parfaitement l'alignement des axes visuels avec les axes de mesure et de stimulation du système 2, 32, 42. Autrement dit, grâce à ces différents paramètres, on fait en sorte de régler les axes de mesure et de stimulation pour qu'ils passent par les centres de rotation CRO des deux yeux.

La mise en place de l'alignement étant faite, il est possible de lancer une nouvelle mesure Mn qui permet d'obtenir les paramètres suivants :
- Sph Vnm (sphère mesurée en position Pn)
- Cyl Vnm (cylindre mesuré en position Pn)
- Axe Vnm (axe du cylindre mesuré en position Pn)
- ½ IPD Gvpm (option) (demi-écart pupillaire gauche mesuré en position Pn)
- ½ IPD Dvpm (option) (demi-écart pupillaire droit mesuré en position Pn).
On peut déterminer les paramètres pour la position Vn de la manière suivante :
- Sph Vn = Sph Vnm
- Cyl Vn = Cyl Vnm
- Axe Vn = Axe Vnm

Néanmoins, on peut déterminer plus précisément la compensation à appliquer en position n de la manière suivante. En effet, la plupart des réfractomètres objectifs ne permettent pas de réaliser une mesure suffisamment précise de la réfraction du sujet, en tout cas au niveau de précision requis pour compenser de manière optimale le défaut du sujet.

Pour ce faire, on saisit en entrée les paramètres Sph VL ,Cyl VL ,Axe VL, Add (qui ont étés obtenus de manière précise, généralement par une mesure subjective).

On applique ensuite les formules suivantes :
- Sph Vn = Sph VL + (Sph VLm- Sph Vnm)
- Cyl Vn = Cyl VL + (Cyl VLm - Cyl Vnm)
- Axe Vn = Axe VL + (AxeVLm - Axe Vnm)

On en déduit ainsi au moins une autre mesure pour la position Pn.

Afin d'obtenir d'autres mesures correspondant à d'autres couples angle d'abaissement du regard et valeur de proximité, on peut naturellement réitérer le procédé de mesure décrit ci-dessus.

Selon une première variante, un calcul d'interpolation et/ou d'extrapolation peut permettre de déduire d'autres mesures à partir d'un ensemble d'au moins deux mesures effectives de réfraction correspondant à deux couples différents (angle d'abaissement du regard, et valeur de proximité).

Selon une autre variante, on utilise un calcul connu de l'homme du métier, basé sur une décomposition vectorielle en polynômes de Zernike, pour déterminer deux composantes J0 et J45 représentatives d'une mesure de l'astigmatisme (cf E.A.H. Malien et al., « Clinical évaluation of the Shin-Nippon SRW-5000 Autorefractor in adults », Ophtal. Physiol. Opt. Vol. 21, No. 2, pp. 101-107, 2001).

Le dispositif d'ensemble peut être consacré à une mesure binoculaire qui assure un bon maintien des paramètres physiologiques d'accommodation et de convergence. La mesure peut cependant être réalisée de façon monoculaire en forçant l'axe de visée de la cible à reproduire la position équivalente binoculaire, cette position fournissant des conditions de mesures proches mais non équivalentes à une mesure binoculaire. Dans les deux cas, on se place dans un alignement des axes de mesure et de stimulation avec le ou les centres de rotation optique (CRO) de l'oeil ou des yeux.

### Mise en oeuvre

De préférence, on utilise les paramètres de réglage suivants.

Dans les conditions contrôlées liées à un verre, paramétrable ou non, la proximité à donner à la cible serait de 2.5 dioptries en VP. Cependant il est intéressant de noter que cette valeur peut être modifiée sur une plage comprise entre 1 et 5 dioptries et réglée de façon à correspondre à une mesure effectuée sur le sujet. On peut viser un angle d'abaissement du regard compris entre 0 et 40 degrés pour la composante de l'angle d'abaissement du regard provenant des yeux seuls. Si l'on ne mesure que deux positions (VL/VP par exemple), on choisit de préférence les angles d'abaissement de 0 et 36 degrés (ou tout angle d'abaissement correspondant aux variantes de la lentille correctrice multifocale ou progressive que l'on souhaite configurer selon la longueur de progression). L'inclinaison de la tête 30 peut aussi varier, l'angle d'inclinaison du plan de Francfort étant compris entre -30 et 30 degrés. Ainsi la dynamique de mesure s'étend sur une gamme d'angle d'abaissement du regard comprise entre -30 et 70 degrés.

La valeur du paramètre de demi-écart pupillaire (1/2 IPD) peut être adaptée de façon symétrique ou non (gérée par demi-IPD) et à partir d'une mesure extérieure ou à partir d'un retour du réglage de l'appareil en VL lors de l'alignement.

L'invention est particulièrement adaptée pour toute personne qui pratique des mesures ophtalmologiques par réfraction et qui désire proposer ou effectuer des mesures en suivant les abaissements physiologiques de la tête et des yeux du sujet. Le dispositif de l'invention peut être utilisé par un optométriste ou un ophtalmologiste, ou encore par un opticien pour déterminer les paramètres de personnalisation d'un verre de lunettes. Le dispositif et le procédé et l'invention peuvent servir à définir les moyens nécessaires à la prescription d'une lentille correctrice multi-focale ou progressive.

## Revendications

1. Dispositif de détermination d'au moins un paramètre de réfraction oculaire objective d'un sujet en fonction d'une pluralité de directions de regard du sujet, ledit dispositif comprenant :
- des moyens de mesure ophtalmologique (2, 32, 42, 52) d'au moins un paramètre de réfraction oculaire objective d'un sujet, lesdits moyens de mesure ophtalmologique (2, 32, 42, 52) ayant au moins un axe optique de mesure (5, 35, 45) destiné(s) à être aligné(s) sur l'axe oculaire droit (21, 22), respectivement gauche (11, 12), du sujet, et
- des moyens de stimulation visuelle (3, 33, 43, 53) destinés à stimuler l'accommodation et la convergence visuelle du sujet dans au moins un axe optique de stimulation superposé avec ledit au moins un axe optique de mesure (5, 35, 45), lesdits moyens de stimulation visuelle (3, 33, 43, 53) ayant une proximité variable entre une première valeur de proximité et au moins une autre valeur de proximité ;
ledit dispositif comprend :
- des moyens opto-mécaniques d'alignement (LM1, LM2, 13, 14, 23, 24, 16, 26, 7, 37, 47), lesdits moyens opto-mécaniques d'alignement étant aptes à effectuer un premier alignement optique dudit au moins un axe optique de mesure (5, 35, 45) sur l'axe oculaire droit, et/ou respectivement gauche dans une première position de mesure, ladite première position de mesure correspondant à un premier couple formé d'un premier angle d'abaissement du regard associé à une première valeur de proximité, et simultanément à une première valeur d'angle de convergence (BETA, BETA1, BETA2) résultant de ladite première valeur de proximité, de manière à effectuer une première mesure d'au moins un paramètre de réfraction oculaire objective dudit sujet pour ledit premier couple angle d'abaissement du regard et valeur de proximité associé à cette première valeur d'angle de convergence (BETA, BETA1, BETA2), et
- lesdits moyens opto-mécaniques d'alignement (LM1, LM2, 13, 14, 23, 24, 16, 26, 7, 37, 47) étant aptes à effectuer au moins un autre alignement optique dudit au moins un axe optique de mesure (5, 35, 45) sur l'axe oculaire droit, et/ou respectivement gauche, dans au moins une autre position de mesure correspondant à un autre couple formé d'un autre angle d'abaissement du regard associée à une autre valeur de proximité, et simultanément à une autre valeur d'angle de convergence (BETA, BETA1, BETA2) résultant de ladite autre valeur de proximité, de manière à effectuer au moins une deuxième mesure dudit au moins un paramètre de réfraction oculaire objective dudit sujet pour ledit au moins un autre couple angle d'abaissement du regard et valeur de proximité associé à cette autre valeur d'angle de convergence (BETA, BETA1, BETA2).

2. Dispositif de détermination d'au moins un paramètre de réfraction oculaire objective selon la revendication 1, dans lequel ledit premier angle d'abaissement du regard est un angle nul par rapport à l'horizontale (11, 12) et ladite première valeur de proximité est comprise entre 0 et 10 dioptries.

3. Dispositif de détermination d'au moins un paramètre de réfraction oculaire objective selon la revendication 1 ou la revendication 2, dans lequel lesdits moyens opto-mécaniques d'alignement comprennent au moins une lame semi-réfléchissante (13, 14, 23, 24) et au moins un obturateur, ladite au moins une lame semi-réfléchissante (13, 14, 23, 24) étant disposée sur ledit au moins un axe optique de mesure (5, 35, 45) de manière à définir un premier axe secondaire de mesure et au moins un autre axe secondaire de mesure, ledit au moins un obturateur commutant de manière à obturer ledit premier ou ledit au moins un autre axe secondaire de mesure, ledit premier axe secondaire de mesure étant destiné à être aligné sur un axe oculaire (11, 21) orienté suivant un premier angle d'abaissement du regard et ledit au moins un autre axe secondaire de mesure étant destiné à être aligné sur un axe oculaire (12, 22) orienté suivant un autre angle d'abaissement du regard.

4. Dispositif de détermination d'au moins un paramètre de réfraction oculaire objective selon la revendication 1 ou la revendication 2, dans lequel lesdits moyens opto-mécaniques d'alignement comprennent des moyens de déplacement desdits moyens de mesure ophtalmologique (2, 32, 42, 52) et des moyens de guidage mécanique (7, 37, 47) des moyens de déplacement suivant une trajectoire prédéterminée en fonction de l'angle d'abaissement du regard et de l'écartement pupillaire d'un sujet de référence.

5. Dispositif de détermination d'au moins un paramètre de réfraction oculaire objective selon la revendication 4, dans lequel lesdits moyens de guidage mécanique (7, 37, 47) comprennent un premier rail de guidage (47) en forme de portion d'une première sphère (25) et/ou un deuxième rail de guidage (37) en forme de portion d'une deuxième sphère (15), la première sphère (25) étant centrée sur le centre de rotation optique de l'oeil mesuré droit (20) et/ou respectivement la deuxième sphère (15) étant centrée sur le centre de rotation optique de l'oeil mesuré gauche (10) du sujet.

6. Dispositif de détermination d'au moins un paramètre de réfraction oculaire objective selon l'une des revendications 1 à 5, comprenant en outre des moyens de détection de la direction et de la position du regard dudit sujet dans ladite première position de mesure et dans ladite au moins une autre position de mesure, et des moyens d'asservissement pour asservir ledit premier alignement optique dudit au moins un axe optique de mesure (5, 35, 45) sur l'axe oculaire droit, et/ou respectivement gauche dans ladite première position de mesure et pour asservir ledit au moins un autre alignement optique dudit au moins un axe optique de mesure (5, 35, 45) sur l'axe oculaire droit, et/ou respectivement gauche, dans ladite au moins une autre position de mesure.

7. Dispositif de détermination d'au moins un paramètre de réfraction oculaire objective selon l'une des revendications 1 à 6, dans lequel l'angle d'abaissement du regard (ALPHA) par rapport à l'horizontale est compris entre -30 degrés et +70 degrés.

8. Dispositif de détermination d'au moins un paramètre de réfraction oculaire objective selon l'une des revendications 1 à 7, dans lequel la valeur de proximité desdits moyens de stimulation visuelle (3, 33, 43, 53) est comprise entre -3 et +10 dioptries en proximité.

9. Dispositif de détermination d'au moins un paramètre de réfraction oculaire objective selon l'une des revendications 1 à 8, dans lequel le dispositif comprend une première position prédéterminée (32) pour ledit au moins un premier angle d'abaissement du regard et une deuxième position prédéterminée (52) pour ledit au moins un autre angle d'abaissement du regard.

10. Dispositif de détermination d'au moins un paramètre de réfraction oculaire objective selon l'une des revendications 1 à 9, dans lequel le dispositif de mesure comprend des premiers moyens de mesure ophtalmologique (42) de la réfraction oculaire de l'oeil droit (20), des premiers moyens de stimulation visuelle (43) de l'oeil droit (20), des deuxièmes moyens de mesure ophtalmologique (32) de la réfraction oculaire objective de l'oeil gauche (10), et des deuxièmes moyens de stimulation visuelle (33) de l'oeil gauche (10), lesdits premiers et deuxièmes moyens de stimulation (33, 43), ayant une même valeur de proximité (Di) pour un même angle d'abaissement du regard.

11. Dispositif de détermination d'au moins un paramètre de réfraction oculaire objective selon l'une des revendications 1 à 10 comprenant au moins un système optique (16, 26) apte à compenser au moins l'un des défauts de réfraction suivants : un défaut de sphère, de cylindre et d'axe, et/ou d'aberrations d'ordre supérieur pour chaque oeil mesuré (10, 20).

12. Procédé de détermination d'au moins un paramètre de réfraction oculaire objective en fonction d'une pluralité de directions de regard d'un sujet à l'aide du dispositif selon l'une des revendications 1 à 11, ledit procédé comprenant les étapes suivantes :
- envoi d'une première stimulation visuelle (3, 33, 43) pour une première valeur de proximité D0 et suivant un premier axe optique de stimulation en direction de l'oeil droit (20), et/ou respectivement gauche (10), d'un sujet ayant un premier angle d'abaissement du regard (ALPHA), de manière à stimuler la convergence et l'accommodation visuelle du sujet pour un premier couple angle d'abaissement du regard et valeur de proximité associé simultanément à une première valeur d'angle de convergence (BETA, BETA1, BETA2) résultant de ladite première valeur de proximité ;
- alignement de l'axe optique de mesure (5, 35, 45) des moyens de mesure ophtalmologique sur l'axe oculaire droit (21), et/ou respectivement sur l'axe oculaire gauche (11) d'un sujet, ledit axe oculaire droit (21) respectivement gauche (11) présentant un premier angle d'abaissement du regard pour une première valeur de proximité et simultanément cette première valeur d'angle de convergence (BETA, BETA1, BETA2) ;
- acquisition d'une première mesure ophtalmologique de réfraction oculaire objective de l'oeil droit, respectivement gauche, dudit sujet pour ledit premier couple angle d'abaissement du regard et valeur de proximité associé à cette première valeur d'angle de convergence (BETA, BETA1, BETA2) ;
- envoi d'une deuxième stimulation visuelle pour au moins un autre couple angle d'abaissement du regard et valeur de proximité en direction de l'oeil droit, et/ou respectivement gauche, d'un sujet ayant au moins un autre angle d'abaissement du regard, de manière à modifier la stimulation de la convergence et de l'accommodation visuelle du sujet pour au moins un autre couple angle d'abaissement du regard et valeur de proximité associé simultanément à une autre valeur d'angle de convergence (BETA, BETA1, BETA2) résultant de ladite autre valeur de proximité,
- alignement de l'axe optique de mesure (5, 35, 45) des moyens de mesure ophtalmologique sur l'axe oculaire droit (22), et/ou respectivement sur l'axe oculaire gauche (12) d'un sujet, ledit axe oculaire droit (22) respectivement gauche (12) présentant un autre angle d'abaissement du regard pour une autre valeur de proximité et simultanément cette autre valeur d'angle de convergence (BETA, BETA1, BETA2) ;
- acquisition d'au moins une deuxième mesure ophtalmologique de réfraction oculaire objective de l'oeil droit, respectivement gauche, d'un sujet pour ledit au moins un autre couple angle d'abaissement du regard et proximité associé à cette autre valeur d'angle de convergence (BETA, BETA1, BETA2) ;
- calcul d'une valeur corrigée de la réfraction oculaire objective de l'oeil droit, et/ou respectivement gauche, d'un sujet suivant l'angle d'abaissement du regard du sujet en fonction desdites première mesure ophtalmologique de réfraction oculaire objective pour ledit premier couple angle d'abaissement du regard et proximité associé à cette première valeur d'angle de convergence (BETA, BETA1, BETA2) et au moins une autre mesure ophtalmologique de réfraction oculaire objective pour ledit au moins un autre couple angle d'abaissement du regard et proximité associé à cette autre valeur d'angle de convergence (BETA, BETA1, BETA2).

13. Procédé de mesure de la cinétique de convergence d'un sujet comprenant les étapes du procédé de la revendication 12, dans lequel la première mesure ophtalmologique de réfraction oculaire objective pour ledit premier couple angle d'abaissement du regard et proximité est effectuée à un premier instant t0, et ladite au moins une autre mesure ophtalmologique de réfraction oculaire objective pour ledit autre couple angle d'abaissement du regard et proximité est effectuée à un autre instant t0+T, les instants t0 et t0+T étant prédéterminés.

14. Procédé de mesure de la cinétique de convergence d'un sujet comprenant les étapes du procédé de la revendication 12 ou 13, dans lequel au moins un paramètre de réfraction oculaire objective mesuré est sélectionné parmi : sphère, cylindre, axe, aberrations d'ordre supérieur, kératométrie et topographie cornéenne ou un différentiel d'un de ces paramètres entre deux angles d'abaissement du regard.

15. Procédé de mesure de la cinétique de convergence d'un sujet comprenant les étapes du procédé de la revendication 14, dans lequel la mesure différentielle objective est utilisée comme donnée d'entrée dans la conception de fabrication d'une lentille correctrice de lunettes progressive ou multifocale.

## Patentansprüche

1. Vorrichtung zur Bestimmung wenigstens eines Parameters der objektiven Augenrefraktion einer Person in Abhängigkeit von mehreren Blickrichtungen der Person, wobei die Vorrichtung umfasst:
- Mittel zur ophthalmologischen Messung (2, 32, 42, 52) wenigstens eines Parameters der objektiven Augenrefraktion einer Person, wobei die Mittel zur ophthalmologischen Messung (2, 32, 42, 52) wenigstens eine optische Messachse (5, 35, 45) aufweisen, die dazu bestimmt ist, auf die rechte (21, 22) bzw. linke (11, 12) Augenachse der Person ausgerichtet zu werden, und
- Mittel zur visuellen Stimulation (3, 33, 43, 53), die dazu bestimmt sind, die visuelle Akkommodation und Konvergenz der Person in wenigstens einer optischen Stimulationsachse, die der wenigstens einen optischen Messachse (5, 35, 45) überlagert ist, zu stimulieren, wobei die Mittel zur visuellen Stimulation (3, 33, 43, 53) eine Nähe aufweisen, die zwischen einem ersten Nähewert und wenigstens einem weiteren Nähewert variabel ist;
wobei die Vorrichtung umfasst:
- optomechanische Ausrichtungsmittel (LM1, LM2, 13, 14, 23, 24, 16, 26, 7, 37, 47), wobei die optomechanischen Ausrichtungsmittel geeignet sind, eine erste optische Ausrichtung der wenigstens einen optischen Messachse (5, 35, 45) auf die rechte und/oder linke Augenachse in einer ersten Messposition durchzuführen, wobei die erste Messposition einem ersten Paar entspricht, das von einem ersten Blicksenkungswinkel gebildet wird, der mit einem ersten Nähewert verknüpft ist, und gleichzeitig mit einem ersten Konvergenzwinkelwert (BETA, BETA1, BETA2), der aus dem ersten Nähewert resultiert, um eine erste Messung wenigstens eines Parameters der objektiven Augenrefraktion der Person für das erste Paar von Blicksenkungswinkel und Nähewert, das mit diesem ersten Konvergenzwinkelwert (BETA, BETA1, BETA2) verknüpft ist, durchzuführen, und
- wobei die optomechanischen Ausrichtungsmittel (LM1, LM2, 13, 14, 23, 24, 16, 26, 7, 37, 47) geeignet sind, wenigstens eine weitere optische Ausrichtung der wenigstens einen optischen Messachse (5, 35, 45) auf die rechte und/oder linke Augenachse in wenigstens einer weiteren Messposition durchzuführen, die einem weiteren Paar entspricht, das von einem weiteren Blicksenkungswinkel gebildet wird, der mit einem weiteren Nähewert verknüpft ist, und gleichzeitig mit einem weiteren Konvergenzwinkelwert (BETA, BETA1, BETA2), der aus dem weiteren Nähewert resultiert, um wenigstens eine zweite Messung des wenigstens einen Parameters der objektiven Augenrefraktion der Person für das wenigstens eine weitere Paar von Blicksenkungswinkel und Nähewert, das mit diesem weiteren Konvergenzwinkelwert (BETA, BETA1, BETA2) verknüpft ist, durchzuführen.

2. Vorrichtung zur Bestimmung wenigstens eines Parameters der objektiven Augenrefraktion nach Anspruch 1, wobei der erste Blicksenkungswinkel ein Winkel null bezüglich der Horizontalen (11, 12) ist und der erste Nähewert zwischen 0 und 10 Dioptrien liegt.

3. Vorrichtung zur Bestimmung wenigstens eines Parameters der objektiven Augenrefraktion nach Anspruch 1 oder Anspruch 2, wobei die optomechanischen Ausrichtungsmittel wenigstens eine halbreflektierende Platte (13, 14, 23, 24) und wenigstens einen Verschluss umfassen, wobei die wenigstens eine halbreflektierende Platte (13, 14, 23, 24) auf der wenigstens einen optischen Messachse (5, 35, 45) so angeordnet ist, dass sie eine erste sekundäre Messachse und wenigstens eine weitere sekundäre Messachse definiert, wobei der wenigstens eine Verschluss so umschaltet, dass er die erste oder die wenigstens eine weitere sekundäre Messachse abdeckt, wobei die erste sekundäre Messachse dazu bestimmt ist, auf eine Augenachse (11, 21) ausgerichtet zu werden, die gemäß einem ersten Blicksenkungswinkel ausgerichtet ist, und die wenigstens eine weitere sekundäre Messachse dazu bestimmt ist, auf eine Augenachse (12, 22) ausgerichtet zu werden, die gemäß einem weiteren Blicksenkungswinkel ausgerichtet ist.

4. Vorrichtung zur Bestimmung wenigstens eines Parameters der objektiven Augenrefraktion nach Anspruch 1 oder Anspruch 2, wobei die optomechanischen Ausrichtungsmittel Mittel zur Verlagerung der Mittel zur ophthalmologischen Messung (2, 32, 42, 52) und Mittel zur mechanischen Führung (7, 37, 47) Verlagerungsmittel entlang einer vorbestimmten Trajektorie in Abhängigkeit von dem Blicksenkungswinkel und von der Pupillendistanz einer Referenzperson umfassen.

5. Vorrichtung zur Bestimmung wenigstens eines Parameters der objektiven Augenrefraktion nach Anspruch 4, wobei die Mittel zur mechanischen Führung (7, 37, 47) eine erste Führungsschiene (47) in Form eines Abschnitts einer ersten Kugel (25) und/oder eine zweite Führungsschiene (37) in Form eines Abschnitts einer zweiten Kugel (15) umfasst, wobei die erste Kugel (25) auf dem optischen Rotationsmittelpunkt des gemessenen rechten Auges (20) zentriert ist und/oder die zweite Kugel (15) auf dem optischen Rotationsmittelpunkt des gemessenen linken Auges (10) der Person zentriert ist.

6. Vorrichtung zur Bestimmung wenigstens eines Parameters der objektiven Augenrefraktion nach einem der Ansprüche 1 bis 5, welche außerdem Mittel zur Erkennung der Richtung und der Position des Blickes der Person in der ersten Messposition und in der wenigstens einen weiteren Messposition umfasst, sowie Regelungsmittel zum Regeln der ersten optischen Ausrichtung der wenigstens einen optischen Messachse (5, 35, 45) auf die rechte und/oder linke Augenachse in der ersten Messposition und zum Regeln der wenigstens einen weiteren optischen Ausrichtung der wenigstens einen optischen Messachse (5, 35, 45) auf die rechte und/oder linke Augenachse in der wenigstens einen weiteren Messposition.

7. Vorrichtung zur Bestimmung wenigstens eines Parameters der objektiven Augenrefraktion nach einem der Ansprüche 1 bis 6, wobei der Blicksenkungswinkel (ALPHA) bezüglich der Horizontalen zwischen -30 Grad und +70 Grad liegt.

8. Vorrichtung zur Bestimmung wenigstens eines Parameters der objektiven Augenrefraktion nach einem der Ansprüche 1 bis 7, wobei der Nähewert der Mittel zur visuellen Stimulation (3, 33, 43, 53) zwischen -3 und +10 Dioptrien in der Nähe liegt.

9. Vorrichtung zur Bestimmung wenigstens eines Parameters der objektiven Augenrefraktion nach einem der Ansprüche 1 bis 8, wobei die Vorrichtung eine erste vorbestimmte Position (32) für den wenigstens einen ersten Blicksenkungswinkel und eine zweite vorbestimmte Position (52) für den wenigstens einen weiteren Blicksenkungswinkel umfasst.

10. Vorrichtung zur Bestimmung wenigstens eines Parameters der objektiven Augenrefraktion nach einem der Ansprüche 1 bis 9, wobei die Messvorrichtung erste Mittel zur ophthalmologischen Messung (42) der Augenrefraktion des rechten Auges (20), erste Mittel zur visuellen Stimulation (43) des rechten Auges (20), zweite Mittel zur ophthalmologischen Messung (32) der objektiven Augenrefraktion des linken Auges (10) und zweite Mittel zur visuellen Stimulation (33) des linken Auges (10), wobei die ersten und zweiten Stimulationsmittel (33, 43) für denselben Blicksenkungswinkel denselben Nähewert (Di) aufweisen.

11. Vorrichtung zur Bestimmung wenigstens eines Parameters der objektiven Augenrefraktion nach einem der Ansprüche 1 bis 10, welche wenigstens ein optisches System (16, 26) umfasst, das in der Lage ist, wenigstens einen der folgenden Refraktionsfehler zu kompensieren: Sphären-, Zylinder- und Achsenfehler und/oder Aberrationen höherer Ordnung für jedes gemessene Auge (10, 20).

12. Verfahren zur Bestimmung wenigstens eines Parameters der objektiven Augenrefraktion in Abhängigkeit von mehreren Blickrichtungen einer Person mithilfe der Vorrichtung nach einem der Ansprüche 1 bis 11, wobei das Verfahren die folgenden Schritte umfasst:
- Senden einer ersten visuellen Stimulation (3, 33, 43) für einen ersten Nähewert D0 und entlang einer ersten optischen Stimulationsachse in Richtung des rechten (20) und/oder linken Auges (10) einer Person mit einem ersten Blicksenkungswinkel (ALPHA), um die visuelle Konvergenz und Akkommodation der Person für ein erstes Paar von Blicksenkungswinkel und Nähewert zu stimulieren, das gleichzeitig mit einem ersten Konvergenzwinkelwert (BETA, BETA1, BETA2) verknüpft ist, der aus dem ersten Nähewert resultiert;
- Ausrichtung der optischen Messachse (5, 35, 45) der Mittel zur ophthalmologischen Messung auf die rechte Augenachse (21) und/oder auf die linke Augenachse (11) einer Person, wobei die rechte (21) bzw. linke Augenachse (11) einen ersten Blicksenkungswinkel für einen ersten Nähewert und gleichzeitig diesen ersten Konvergenzwinkelwert (BETA, BETA1, BETA2) aufweist;
- Erfassung eines ersten ophthalmologischen Messwertes der objektiven Augenrefraktion des rechten bzw. linken Auges der Person für das erste Paar von Blicksenkungswinkel und Nähewert, das mit diesem ersten Konvergenzwinkelwert (BETA, BETA1, BETA2) verknüpft ist;
- Senden einer zweiten visuellen Stimulation für wenigstens ein weiteres Paar von Blicksenkungswinkel und Nähewert in Richtung des rechten und/oder linken Auges einer Person mit wenigstens einem weiteren Blicksenkungswinkel, um die Stimulation der visuellen Konvergenz und Akkommodation der Person für wenigstens ein weiteres Paar von Blicksenkungswinkel und Nähewert zu modifizieren, das gleichzeitig mit einem weiteren Konvergenzwinkelwert (BETA, BETA1, BETA2) verknüpft ist, der aus dem weiteren Nähewert resultiert;
- Ausrichtung der optischen Messachse (5, 35, 45) der Mittel zur ophthalmologischen Messung auf die rechte Augenachse (22) und/oder auf die linke Augenachse (12) einer Person, wobei die rechte (22) bzw. linke Augenachse (12) einen weiteren Blicksenkungswinkel für einen weiteren Nähewert und gleichzeitig diesen weiteren Konvergenzwinkelwert (BETA, BETA1, BETA2) aufweist;
- Erfassung wenigstens eines zweiten ophthalmologischen Messwertes der objektiven Augenrefraktion des rechten bzw. linken Auges einer Person für das wenigstens eine weitere Paar von Blicksenkungswinkel und Nähe, das mit diesem weiteren Konvergenzwinkelwert (BETA, BETA1, BETA2) verknüpft ist;
- Berechnung eines korrigierten Wertes der objektiven Augenrefraktion des rechten und/oder linken Auges einer Person gemäß dem Blicksenkungswinkel der Person in Abhängigkeit von dem ersten ophthalmologischen Messwert der objektiven Augenrefraktion für das erste Paar von Blicksenkungswinkel und Nähe, das mit diesem ersten Konvergenzwinkelwert (BETA, BETA1, BETA2) verknüpft ist, und wenigstens einem weiteren ophthalmologischen Messwert der objektiven Augenrefraktion für das wenigstens eine weitere Paar von Blicksenkungswinkel und Nähe, das mit diesem weiteren Konvergenzwinkelwert (BETA, BETA1, BETA2) verknüpft ist.

13. Verfahren zur Messung der Kinetik der Konvergenz einer Person, welches die Schritte des Verfahrens von Anspruch 12 umfasst, wobei die erste ophthalmologische Messung der objektiven Augenrefraktion für das erste Paar von Blicksenkungswinkel und Nähe zu einem ersten Zeitpunkt t0 durchgeführt wird und die wenigstens eine weitere ophthalmologische Messung der objektiven Augenrefraktion für das weitere Paar von Blicksenkungswinkel und Nähe zu einem weiteren Zeitpunkt t0+T durchgeführt wird, wobei die Zeitpunkte t0 und t0+T vorbestimmt sind.

14. Verfahren zur Messung der Kinetik der Konvergenz einer Person, welches die Schritte des Verfahrens von Anspruch 12 oder 13 umfasst, wobei wenigstens ein gemessener Parameter der objektiven Augenrefraktion ausgewählt ist aus: Sphäre, Zylinder, Achse, Aberrationen höherer Ordnung, Keratometrie und Hornhauttopographie oder eine Differenz eines dieser Parameter zwischen zwei Blicksenkungswinkeln.

15. Verfahren zur Messung der Kinetik der Konvergenz einer Person, welches die Schritte des Verfahrens von Anspruch 14 umfasst, wobei die objektive differentielle Messung als Eingabegröße beim Entwurf für die Herstellung eines Gleitsicht- oder multifokalen Brillenkorrekturglases verwendet wird.

## Claims

1. A device for determining at least one objective ocular refraction parameter of a subject as a function of a plurality of gaze directions of the subject, said device comprising:
- ophthalmological means (2, 32, 42, 52) for measuring at least one objective ocular refraction parameter of a subject, said ophthalmological measuring means (2, 32, 42, 52) having at least one measuring optical axis (5, 35, 45) that is/are intended to be aligned with the right ocular axis (21, 22) and left ocular axis (11, 12) of the subject, respectively; and
- visual stimulating means (3, 33, 43, 53) intended to stimulate the accommodation and visual convergence of the subject on at least one stimulating optical axis superposed on said at least one measuring optical axis (5, 35, 45), said visual stimulating means (3, 33, 43, 53) having a proximity that may be varied between a first proximity value and at least another proximity value;
said device comprises:
- opto-mechanical aligning means (LM1, LM2, 13, 14, 23, 24, 16, 26, 7, 37, 47), said opto-mechanical aligning means being able to carry out a first optical alignment of said at least one measuring optical axis (5, 35, 45) with the right and/or left ocular axis, respectively, in a first measuring position, said first measuring position corresponding to a first pair made up of a first gaze declination angle associated with a first proximity value, and simultaneously at a first value of gaze convergence angle (BETA, BETA1, BETA2) resulting from said first proximity value, so as to carry out a first measurement of at least one objective ocular refraction parameter of said subject for said first gaze declination angle and proximity value pair associated with said first value of gaze convergence angle; and
- said opto-mechanical aligning means (LM1, LM2, 13, 14, 23, 24, 16, 26, 7, 37, 47) being able to carry out at least another optical alignment of said at least one measuring optical axis (5, 35, 45) with the right and/or left ocular axis, respectively, in at least another measuring position corresponding to another pair made up of another gaze declination angle associated with another proximity value and simultaneously at another value of gaze convergence angle (BETA, BETA1, BETA2) resulting from said another proximity value, so as to carry out at least one second measurement of said at least one objective ocular refraction parameter of said subject for said at least another gaze declination angle and proximity value pair associated with said another value of gaze convergence angle (BETA, BETA1, BETA2).

2. The device for determining at least one objective ocular refraction parameter according to claim 1, in which said first gaze declination angle is an angle of zero relative to the horizontal (11, 12) and said first proximity value is comprised between 0 and 10 diopters.

3. The device for determining at least one objective ocular refraction parameter according to claim 1 or claim 2, in which said opto-mechanical aligning means comprise at least one semi-reflective plate (13, 14, 23, 24) and at least one shutter, said at least one semi-reflective plate (13, 14, 23, 24) being placed on said at least one measuring optical axis (5, 35, 45) so as to define a first secondary measuring axis and at least another secondary measuring axis, said at least one shutter switching so as to block said first or at least another secondary measuring axis, said first secondary measuring axis being intended to be aligned with an ocular axis (11, 21) oriented along a first gaze declination angle and said and at least another secondary measuring axis being intended to be aligned with an ocular axis (12, 22) oriented along another gaze declination angle.

4. The device for determining at least one objective ocular refraction parameter according to claim 1 or claim 2, in which said opto-mechanical aligning means comprise means for moving said ophthalmological measuring means (2, 32, 42, 52) and means (7, 37, 47) for mechanically guiding the moving means along a preset trajectory depending on the gaze declination angle and the pupillary distance of a reference subject.

5. The device for determining at least one objective ocular refraction parameter according to claim 4, in which said mechanical guiding means (7, 37, 47) comprise a first guiding rail (47) taking the form of a portion of a first sphere (25) and/or a second guiding rail (37) taking the form of a portion of a second sphere (15), the first sphere (25) being centered on the optical center of rotation of the measured right eye (20) and/or the second sphere (15) being centered on the optical center of rotation of the measured left eye (10) of the subject, respectively.

6. The device for determining at least one objective ocular refraction parameter according to any one of claims 1 to 5, furthermore comprising means for detecting the direction and the position of the gaze of said subject in said first measuring position and in said at least another measuring position, and controlling means for aligning said first optical alignment of said at least one measuring optical axis (5, 35, 45) with the right and/or left ocular axis, respectively, in said first measuring position and for aligning said at least another optical alignment of said at least one measuring optical axis (5, 35, 45) with the right and/or left ocular axis, respectively, in said at least another measuring position.

7. The device for determining at least one objective ocular refraction parameter according to any one of claims 1 to 6, in which the gaze declination angle (ALPHA) relative to the horizontal is comprised between -30 degrees and +70 degrees.

8. The device for determining at least one objective ocular refraction parameter according to any one of claims 1 to 7, in which the proximity value of said visual stimulating means (3, 33, 43, 53) is comprised between -3 and +10 diopters in proximity.

9. The device for determining at least one objective ocular refraction parameter according to any one of claims 1 to 8, in which the device comprises a first preset position (32) for said at least one first gaze declination angle and a second preset position (52) for said at least another gaze declination angle.

10. The device for determining at least one objective ocular refraction parameter according to any one of claims 1 to 9, in which the measuring device comprises first ophthalmological means (42) for measuring the ocular refraction of the right eye (20), first means (43) for visually stimulating the right eye (20), second ophthalmological means (32) for measuring the objective ocular refraction of the left eye (10), and second means (33) for visually stimulating the left eye (10), said first and second stimulating means (33, 43) having a given proximity value (Di) for a given gaze declination angle.

11. The device for determining at least one objective ocular refraction parameter according to any one of claims 1 to 10, comprising at least one optical system (16, 26) able to correct for at least one of the following refractive errors: a sphere, cylinder and axis error, and/or higher order aberrations for each measured eye (10, 20).

12. A method for determining at least one objective ocular refraction parameter as a function of a plurality of gaze directions of a subject using a device according to any one of claims 1 to 11, said method comprising the following steps:
- delivering a first visual stimulation (3, 33, 43) for a first proximity value D0 and along a first stimulating optical axis in the direction of the right eye (20) and/or left eye (10), respectively, of a subject having a first gaze declination angle (ALPHA), so as to stimulate the convergence and visual accommodation of the subject for a first gaze declination angle and proximity value pair associated simultaneously with a first value of gaze convergence angle (BETA, BETA1, BETA2) resulting from said first proximity value;
- aligning the measuring optical axis (5, 35, 45) of the ophthalmological measuring means with the right ocular axis (21) and/or with the left ocular axis (11) of a subject, respectively, said right ocular axis (21) and left ocular axis (11) respectively having a first gaze declination angle for a first proximity value and simultaneously said first value of gaze convergence angle (BETA, BETA1, BETA2);
- acquiring a first objective ophthalmological ocular refraction measurement for the right eye and left eye of said subject, respectively, for said first gaze declination angle and proximity value pair associated with said first value of gaze convergence angle (BETA, BETA1, BETA2);
- delivering a second visual stimulation for at least another gaze declination angle and proximity value pair in the direction of the right and/or left eye, respectively, of a subject having at least another gaze declination angle, so as to modify the stimulation of the convergence and of the visual accommodation of the subject for at least another gaze declination angle and proximity value pair associated simultaneously with another value of gaze convergence angle (BETA, BETA1, BETA2) resulting from said another proximity value;
- aligning the measuring optical axis (5, 35, 45) of the ophthalmological measuring means with the right ocular axis (22) and/or the left ocular axis (12) of a subject, respectively, said right ocular axis (22) and left ocular axis (12) respectively having another gaze declination angle for another proximity value and simultaneously said another value of gaze convergence angle (BETA, BETA1, BETA2);
- acquiring at least one second objective ophthalmological ocular refraction measurement for the right eye and left eye of a subject, respectively, for said at least another gaze declination angle and proximity pair associated with said another value of gaze convergence angle (BETA, BETA1, BETA2); and
- calculating a corrected value of the objective ocular refraction of the right eye and/or left eye of a subject, respectively, as a function of the gaze declination angle of the subject and depending on said first objective ophthalmological ocular refraction measurement for said first gaze declination angle and proximity pair associated with said first value of gaze convergence angle (BETA, BETA1, BETA2) and on said at least another objective ophthalmological ocular refraction measurement for said at least another gaze declination angle and proximity pair associated with said another value of gaze convergence angle (BETA, BETA1, BETA2).

13. A method for measuring the convergence kinetics of a subject, comprising the steps of the method of claim 12, in which the first objective ophthalmological ocular refraction measurement for said first gaze declination angle and proximity pair is carried out at a first time t0, and said at least another objective ophthalmological ocular refraction measurement for said other gaze declination angle and proximity pair is carried out at another time t0+T, the times t0 and t0+T being preset.

14. A method for measuring the convergence kinetics of a subject, comprising the steps of the method of claim 12 or 13, in which at least one measured objective ocular refraction parameter is selected from: sphere, cylinder, axis, higher-order aberrations, keratometry and corneal topography or a measurement of one of these parameters differentiated between two gaze declination angles.

15. A method for measuring the convergence kinetics of a subject, comprising the steps of the method of claim 14, in which the objective differential measurement is used as input data in the manufacturing design of a corrective progressive or multifocal lens for a pair of spectacles.
